# EUROPÄISCHE PATENTSCHRIFT

(11) **EP 3 043 840 B1**
(45) Veröffentlichungstag und Bekanntmachung des Hinweises auf die Patenterteilung: **16.08.2017**
(21) Anmeldenummer: 13762439.1
(22) Anmeldetag: 09.09.2013
(51) Int. Cl.: A61M 5/20, A61M 5/315

(54) **INJEKTIONSVORRICHTUNG MIT EINEM BETÄTIGUNGSKNOPF, DESSEN BETÄTIGUNG EINE DREHBEWEGUNG BEWIRKT**
INJECTION DEVICE HAVING AN ACTUATING KNOB, ACTUATION OF WHICH EFFECTS A ROTARY MOVEMENT
DISPOSITIF D'INJECTION COMPORTANT UN BOUTON D'ACTIONNEMENT DONT L'ACTIONNEMENT PROVOQUE UN MOUVEMENT DE ROTATION

(43) Veröffentlichungstag der Anmeldung: 20.07.2016
(73) Patentinhaber: Tecpharma Licensing AG, 3401 Burgdorf (CH)
(72) Erfinder: SCHENKER, Susanne, 4912 Aarwangen (CH); STREIT, Ursina, 3322 Schönbühl (CH); HIRSCHEL, Jürg, 3007 Bern (CH)
(86) Internationale Anmeldenummer: PCT/EP2013/068647
(87) Internationale Veröffentlichungsnummer: WO 2015/032455

(56) Entgegenhaltungen:
- WO-A1-01/72361
- WO-A1-2013/119132
- WO-A2-02/092153
- CH-A2- 705 155
- US-A1- 2012 197 213

## Beschreibung

Die Erfindung liegt auf dem Gebiet der Injektionsvorrichtungen zum Verabreichen eines flüssigen Produkts, insbesondere eines Medikaments, wie z. B. Insulin zur Diabetestherapie. Die Erfindung betrifft eine Antriebsvorrichtung, wie z. B. eine Antriebs- und Dosiervorrichtung für eine solche Injektionsvorrichtung.

Aus dem Stand der Technik, nämlich der WO 2008/031237 A1 ist ein Injektionsgerät mit einer Dosisanzeigetrommel und einer Antriebsfeder bekannt. Die Antriebsfeder ist eine Uhrenfeder, die spiralförmig aus einem bandförmigen Material gewickelt ist. Beim Einstellen der gewünschten Produktdosis wird die Feder mit einer rotatorischen Bewegung gespannt. Für die Dosisausschüttung wird durch Betätigen eines Betätigungsknopfs am proximalen Ende der Vorrichtung eine Kolbenstange der Vorrichtung mit der Feder gekoppelt, wodurch die Feder die in ihr gespeicherte Energie an die Kolbenstange abgeben kann, wodurch die Kolbenstange in Ausschüttrichtung bewegt wird. Zum Einstellen einer neuen Dosis wird die Feder durch Drehen des Dosierknopfs wieder vorgespannt und so weiter. Dies wird so oft wiederholt, bis der Produktbehälter geleert ist.

In den Patentanmeldungen WO 2002/092153 und EP 2644217 wird eine Antriebs- und Dosiervorrichtung beschrieben, wie sie in den beigefügten Figuren 1 bis 5c dargestellt wird. Diese Vorrichtung weist eine Antriebsfeder auf, die im Auslieferungszustand der Antriebs- und Dosiervorrichtung mit soviel Energie vorgespannt ist, dass sie die aus dem Produktbehälter ausschüttbare Produktmenge in mehreren Einzelausschüttungen ausschütten kann. Die Ausschüttfeder treibt ein Rotationsglied an, dessen Rotation bewirkt, dass ein Vortriebsglied den Kolben eines Produktbehälters verschiebt, wodurch das in dem Produktbehälter enthaltene Produkt ausgeschüttet wird. Für die Produktausschüttung wird ein Druckknopf betätigt, wodurch eine geschlossene Kupplung geöffnet wird, wobei die geöffnete Kupplung die Drehung des Rotationsglieds zulässt und die geschlossene Kupplung die Drehung des Rotationsglieds nicht zulässt.

Derartige Injektionsvorrichtungen setzen voraus, dass die vorgespannte Ausschüttfeder ohne weiteres Zutun die Produktausschüttung startet, wenn der Betätigungsknopf bzw. Druckknopf vollständig betätigt ist. Da solche Injektionsvorrichtungen mitunter lange bis zu ihrer Verwendung lagern können, kann es sein, dass der Mechanismus "fest" wird bzw. sich nicht ordnungsgemäß löst, wenn der Betätigungsknopf vollständig gedrückt ist, so dass eine Produktausschüttung z. B. verzögert startet oder erst durch leichtes Klopfen (mechanisches Erschüttern) gegen die Injektionsvorrichtung startet.

Es ist eine Aufgabe der Erfindung eine Antriebsvorrichtung für eine Injektionsvorrichtung anzugeben, die sicherstellt, dass die Produktausschüttung bei betätigtem Betätigungsglied startet.

Die Aufgabe wird durch die Merkmale des Anspruchs 1 gelöst. Vorteilhafte Weiterbildungen ergeben sich aus den abhängigen Ansprüchen, der Beschreibung und den Figuren.

Die Erfindung geht von einer Antriebsvorrichtung, insbesondere einer Antriebs- und Dosiervorrichtung, für eine Injektionsvorrichtung zum Verabreichen eines flüssigen Medikaments oder Produkts aus. Mit der Antriebsvorrichtung kann eine feste oder nicht veränderbare Dosis ausgeschüttet oder eine Dosis eingestellt und anschließend ausgeschüttet werden. Sofern die Antriebsvorrichtung die Einstellung einer, z. B. variablen, Dosis erlaubt, wie z. B. durch Verdrehen eines vom Verwender greifbaren Dosierglieds, kann sie auch als Antriebs- und Dosiervorrichtung bezeichnet werden.

Die Antriebsvorrichtung weist ein Gehäuse auf. Das Gehäuse ist vorzugsweise hülsenförmig und/oder länglich ausgebildet. Das Gehäuse kann sich z. B. entlang seiner Längsachse erstrecken.

Das Gehäuse kann optional einen Produktbehälter aufnehmen oder selbst den Produktbehälter bilden. Das Gehäuse kann ein- oder mehrteilig sein. Z. B. kann das Gehäuse einen proximalen Gehäuseteil bilden, der die Antriebsvorrichtung umfasst oder aufweist. Das Gehäuse kann ferner einen Produktbehälterhalter aufweisen, der den Produktbehälter, wie z. B. eine Karpule, aufnimmt und mit dem Gehäuse oder dem proximalen Gehäuseteil verbunden ist. Diese Verbindung kann derart sein, dass der Produktbehälterhalter und das Gehäuse oder der proximale Gehäuseteil nach dem Verbinden unlösbar, d. h. nur durch Zerstörung von Verbindungselementen lösbar ist. Eine solche Ausführung ist insbesondere bei Einweginjektionsvorrichtungen von Vorteil, die nach dem das in dem Produktbehälter enthaltene Produkt vollständig ausgeschüttet ist, als Ganzes entsorgt werden. Alternativ kann der Produktbehälterhalter auch lösbar an dem Gehäuse befestigt werden, wodurch es möglich ist, die Antriebsvorrichtung gegebenenfalls mehrfach zu verwenden, d. h. einen leeren Produktbehälter gegen einen gefüllten Produktbehälter auszutauschen.

Das Gehäuse dient vorzugsweise dazu, vom Verwender der Vorrichtung ergriffen zu werden. Insbesondere kann das Gehäuse eine im Wesentlichen zylindrische Form aufweisen. Das Gehäuse kann z. B. eine Zeigeeinrichtung, insbesondere ein Fenster aufweisen, mittels der oder durch welches eine aktuell eingestellte Dosis, vorzugsweise von einer Skala eines Dosiseinstelleelements, ablesbar ist.

Die Antriebsvorrichtung kann ein Vortriebsglied aufweisen, dessen distales Ende vorgesehen ist, auf einen Kolben, insbesondere mittelbar oder vorzugsweise unmittelbar zu wirken. Der Kolben kann Teil eines an der Antriebsvorrichtung befestigten oder befestigbaren Produktbehälters, wie z. B. einer Karpule sein. Das Vortriebsglied kann im weiteren Sinne als Kolbenstange bezeichnet werden, wobei das Vortriebsglied nicht notwendigerweise massiv sein muss, sondern auch hohl, wie z. B. hülsenförmig ausgestaltet sein kann. An dem distalen Ende des Vortriebsglieds kann optional ein z. B. drehbarer Flansch gebildet sein, der gegen den Kolben drückt. Allgemein ist es bevorzugt, dass das distale Ende des Vortriebsglieds gegen den Kolben drückt. Das Vortriebsglied ist vorzugsweise relativ zu dem Gehäuse entlang der Längsachse der Antriebsvorrichtung verschiebbar.

Die Antriebsvorrichtung kann ein Widerlager und vorzugsweise eine Führung aufweisen, wobei das Vortriebsglied relativ zu dem Widerlager und vorzugsweise auch relativ zu der Führung in eine Richtung, insbesondere in die distale Richtung, d. h. in die Ausschüttrichtung, bewegbar ist, um eine Ausschüttung des Produkts oder ggf. der oder einer eingestellten Produktdosis zu bewirken. Vorzugsweise kann das Vortriebsglied mittels oder an der Führung, entlang der Längsachse der Antriebsvorrichtung gerade oder axial geführt sein. Insbesondere kann das Vortriebsglied relativ zu dem Widerlager oder/und der Führung oder/und dem Gehäuse drehfest sein. In einer alternativen Ausführungsform kann das Vortriebsglied relativ zu dem Widerlager oder dem Gehäuse kombiniert mit einer Längsbewegung drehbar, d. h. relativ zu dem Widerlager schraubbar sein. Allgemein kann die Führung oder/und das Widerlager von dem Gehäuse, insbesondere einem hülsenförmigen Gehäuseteil oder einem z. B. hülsenförmigen, gehäusefesten Element gebildet werden.

Das Vortriebsglied und die insbesondere von dem Gehäuse gebildete Führung können in einem Eingriff sein, der eine Drehung des Vortriebsglieds relativ zu dem Widerlager oder dem Gehäuse verhindert und eine Axialbewegung, oder eine Schraubbewegung des Vortriebsglieds relativ zu dem Widerlager oder dem Gehäuse erlaubt. Die Führung kann z. B. eine Axialführung oder ein Gewinde mit einer nicht selbsthemmenden Gewindesteigung sein.

Die Führung oder der das Widerlager oder/und die Führung bildende Gehäuseabschnitt, insbesondere eine Innenhülse, kann vorzugsweise das Vortriebsglied umgeben, wie z. B. hülsenförmig umgeben und/oder gehäusefest sein oder von dem Gehäuse gebildet werden. Zwischen diesem hülsenförmigen Gehäuseteil und einem äußeren, vorzugsweise ebenfalls hülsenförmigen Gehäuseteil kann ein Ringspalt gebildet sein, der den Vorteil birgt, dass ein optional vorhandenes Dosisanzeigeelement, insbesondere eine Dosisanzeigetrommel, darin z. B. bewegbar aufgenommen sein kann. Dies bewirkt, dass die Länge der Antriebsvorrichtung gering gehalten werden kann.

Die Antriebsvorrichtung kann mindestens eine, wie z. B. genau eine, zwei oder drei zwischen dem Vortriebsglied oder einem Rotationsglied und dem Widerlager wirkende und insbesondere angeordnete Ausschüttfeder aufweisen. Die mindestens eine Feder kann sich z. B. an dem Vortriebsglied und/oder dem Widerlager abstützen. Beispielsweise kann sich die z. B. einzige Ausschüttfeder mit ihrem distalen Ende an dem Vortriebsglied und mit ihrem proximalen Ende an dem Widerlager abstützen. Insbesondere kann die mindestens eine Ausschüttfeder innerhalb der Führung oder des die Führung bildenden hülsenförmigen Gehäuseteils angeordnet sein. Ist das Vortriebsglied hülsenförmig, kann die mindestens eine Ausschüttfeder innerhalb des Vortriebsglieds angeordnet sein. Alternativ können eine erste Ausschüttfeder und eine zweite Ausschüttfeder insbesondere kinematisch zwischen dem Vortriebsglied und der Führung oder dem die Führung bildenden hülsenförmigen Gehäuseteil angeordnet sein. Die erste Ausschüttfeder kann z. B. die zweite Ausschüttfeder umgeben oder umgekehrt. Insbesondere kann die zweite Ausschüttfeder konzentrisch zur ersten Ausschüttfeder angeordnet sein. Die erste Ausschüttfeder und die zweite Ausschüttfeder können z. B. parallel oder in Serie geschaltet sein. Parallel geschaltete Ausschüttfedern bedeuten insbesondere, dass sich die erste und zweite Ausschüttfeder jeweils mit ihrem distalen Ende an dem Vortriebsglied und jeweils mit ihrem proximalen Ende an dem Widerlager abstützen können. Hierdurch lassen sich die Federkonstanten der ersten und zweiten Ausschüttfeder zu einer Gesamtfederkonstante addieren. In Serie geschaltete Ausschüttfedern bedeutet insbesondere, dass das distale Ende von einer aus erster und zweiter Ausschüttfeder gegen das proximale Ende der anderen aus erster und zweiter Ausschüttfeder drückt, insbesondere unmittelbar oder vorzugsweise mittelbar, wie z. B. über ein Zwischenglied. Z. B. stützt sich die erste Ausschüttfeder an dem Widerlager und dem Zwischenglied und die zweite Ausschüttfeder an dem Zwischenglied und dem Vortriebsglied ab. Beispielsweise kann das distale Ende der ersten Ausschüttfeder distal des proximalen Endes der zweiten Ausschüttfeder angeordnet sein. Aufgrund des Zwischenglieds kann die Federkraft der ersten Feder von ihrem distalen Ende auf das proximale Ende der zweiten Ausschüttfeder übertragen werden. Insbesondere kann das Zwischenglied hülsenförmig sein und in einem Ringspalt zwischen erster und zweiter Ausschüttfeder angeordnet sein. In Serie geschaltete Ausschüttfedern erlauben, über einen verhältnismäßig langen Federweg eine verhältnismäßig gleich bleibende Federkraft.

Z. B. kann die mindestens eine Ausschüttfeder, insbesondere die erste und zweite Ausschüttfeder eine Wendel- oder Schraubenfeder sein, die als Druckfeder oder als Torsionsfeder wirkt. Die mindestens eine Ausschüttfeder ist vorgespannt und wirkt so auf das Vortriebsglied, das sie versucht, das Vortriebsglied relativ zu dem Widerlager in die distale Richtung, d. h. in die Ausschüttrichtung zu verschieben. Die mindestens eine Ausschüttfeder ist im Auslieferungszustand der Antriebs- und Dosiervorrichtung insbesondere mit so viel Energie vorgespannt, dass sie die aus dem Produktbehälter maximal oder insgesamt ausschüttbare Produktmenge, zum Beispiel in mehreren Einzelausschüttungen, d. h. insbesondere in mehreren Ausschüttungen einzelner Produktdosen, ausschütten kann.

Die Antriebsvorrichtung kann, wenn mit ihr eine Dosis einstellbar ist, so ausgestaltet sein, dass nach jeder Einzelausschüttung oder Ausschüttung der Produktdosis, die nächste auszuschüttende Dosis neu eingestellt wird. Die Antriebsvorrichtung kann ein Dosierglied aufweisen, das zum Beispiel als Dosierknopf ausgebildet ist und optional als Dosiseinstellglied bezeichnet werden kann. Im Gegensatz zu Ausführungen, bei denen eine Ausschüttfeder bei jeder Dosiseinstellung neu vorgespannt wird, kann durch die mit der für die Ausschüttung der maximal aus dem Produktbehälter ausschüttbaren Produktmenge erforderlichen Energie vorgespannte Feder eine einfachere Dosiseinstellung erreicht werden, da das für die Dosiseinstellung relativ zu dem Gehäuse drehbare Dosierglied dann einfacher drehbar ist, weil die Feder bei der Dosiseinstellung nicht vorgespannt werden braucht. Dies erhöht den Anwendungskomfort für den Verwender der Vorrichtung.

Die Antriebsvorrichtung kann ferner ein Rotationsglied umfassen, dessen Drehung bewirkt, dass die Feder Energie an das Vortriebsglied abgibt, wodurch das Vortriebsglied in die distale Richtung bewegt wird. Das Rotationsglied übernimmt vorzugsweise die Funktion eines Steuerglieds, wobei die Drehung des Rotationsglieds um einen bestimmten Drehwinkel den Vorschub des Vortriebsglieds um einen bestimmten Ausschütthub bewirkt. Durch selektive Freigabe oder Sperrung einer Drehung des Rotationsglieds relativ zu dem Gehäuse kann der Feder erlaubt werden, dass sie das Vortriebsglied relativ zu dem Widerlager in die distale Richtung bewegen kann oder nicht bewegen kann. Insbesondere kann das Rotationsglied so mit dem Betätigungsglied gekoppelt sein, dass es bei der Betätigung des Betätigungsglieds für eine Produktausschüttung für eine Drehung relativ zu dem Gehäuse freigegeben ist und bei Nichtbetätigung des Betätigungsglieds für eine Rotation relativ zu dem Gehäuse gesperrt ist. Insbesondere kann zwischen Betätigungsglied und Rotationsglied eine Kupplung, insbesondere Ausschüttkupplung, angeordnet sein, welche die Freigabe und Sperrung der Drehung des Rotationsglieds relativ zu dem Gehäuse bewirkt. Die geschlossene Kupplung kann durch Betätigen, insbesondere Drücken des Betätigungsglieds geöffnet werden, wobei die geöffnete Kupplung die - mittels der vorgespannten Feder bewirkbare - Drehung des Rotationsglieds, insbesondere Ausschüttfeder, relativ zu dem Gehäuse freigibt.

Vorteilhaft kann die Kupplung durch Betätigen des Betätigungsglieds die Drehung des Rotationsglieds relativ zu dem Gehäuse freigeben und durch Loslassen des Betätigungsglieds die Drehung des Rotationsglieds relativ zu dem Gehäuse blockieren.

Das Betätigungsglied ist so mit dem Rotationsglied gekoppelt, dass das Betätigen, insbesondere das Drücken des Betätigungsglieds bewirkt, dass sich das Rotationsglied relativ zu dem Gehäuse bereits dann dreht, wenn die Kupplung noch geschlossen ist. Das Betätigungselement ist zwischen einer unbetätigten, d. h. ungedrückten Position, und einer betätigten Position, d. h. gedrückten Position, hin und her verschiebbar, insbesondere entlang der Längsachse der Antriebsvorrichtung. Durch die Verschiebung des Betätigungsglieds aus der unbetätigten in die betätigte Position wird das Rotationsglied relativ zu dem Gehäuse bereits dann gedreht, wenn die Kupplung noch geschlossen ist. Die unbetätigte Position kann auch als Ausgangsposition des Betätigungsglieds bezeichnet werden. Die Kupplung ist geöffnet, wenn das Betätigungsglied in seiner betätigten Position ist. Das Betätigungsglied ist mit dem Rotationsglied über ein Getriebe gekoppelt, welches die Bewegung des Betätigungsglieds entlang der Längsachse L, insbesondere in die distale Richtung, in eine Drehbewegung des Rotationsglieds, insbesondere in die erste Drehrichtung, umwandelt. Durch die Drehung des Rotationsglieds bereits dann, wenn die Kupplung noch geschlossen ist, lässt sich vorteilhaft erreichen, dass die mit der Zeit möglicherweise zueinander festgewordenen Teile gelöst werden, insbesondere durch die Muskelkraft des Verwenders, welche das Betätigungsglied in die distale Richtung drückt.

Bevorzugt ist, dass die durch das Betätigen des Betätigungsglieds bewirkte Drehung des Rotationsglieds relativ zu dem Gehäuse bei geschlossener Kupplung kleiner als 45°, insbesondere kleiner als 20° ist. Vorzugsweise ist die durch das Betätigen des Betätigungsglieds bewirkte Drehung des Rotationsglieds relativ zu dem Gehäuse in die gleiche Drehrichtung, insbesondere in die erste Drehrichtung, alternativ in die entgegensetzte Drehrichtung, insbesondere die zweite Drehrichtung gerichtet, wie die Drehrichtung der mittels der vorgespannten Feder bewirkbaren Drehung des Rotationsglieds. Die mittels der vorgespannten Feder bewirkbare Drehung des Rotationsglieds erfolgt in die erste Drehrichtung.

Insbesondere kann das Betätigungsglied so mit dem Rotationsglied gekoppelt sein, dass das Zurückbewegen des Betätigungsglieds aus seiner betätigten Positionen in seine unbetätigte Position, insbesondere das Loslassen des Betätigungsglieds, bewirkt, dass sich das Rotationsglied relativ zu dem Gehäuse in eine Drehrichtung, insbesondere in die zweite, d. h. der ersten Drehrichtung entgegengesetzte Drehrichtung, dreht, die der Drehrichtung, insbesondere der ersten Drehrichtung, der mittels der vorgespannten Feder bewirkbaren Drehung entgegengesetzt ist. Dieser Effekt kann mittels des oben genannten Getriebes erfolgen. Insbesondere kann das Betätigungsglied von einer Feder, insbesondere einer Rücksetz- oder Kupplungsfeder, die durch das Betätigen des Betätigungsglieds vorgespannt wird, aus seiner betätigten Position in seine unbetätigte Position zurückbewegt werden. Diese Feder kann hierdurch das Rotationsglied, insbesondere über das Getriebe, in die zweite Drehrichtung drehen.

Insbesondere in Ausführungsformen, in denen das Loslassen des Betätigungsglieds bewirkt, dass sich das Rotationsglied relativ zu dem Gehäuse in die zweite Drehrichtung dreht, ist es bevorzugt, dass der Kolben des Produktbehälters bei der Produktausschüttung oder bei der Drehung des Rotationsglieds in die erste Drehrichtung von dem auf ihn in Ausschüttrichtung wirkenden Vortriebsglied in Ausschüttrichtung verschoben wird und dass beim Zurückbewegen des Betätigungsglieds in seine unbetätigte Position das Vortriebsglied entgegen die Ausschüttrichtung relativ zu dem Widerlager bewegt wird, insbesondere soweit, dass der Kolben von dem Druck des Vortriebsglieds entlastet wird. Durch die Drehung des Rotationsglieds in die zweite Drehrichtung wird bewirkt, dass das Vortriebsglied ein zumindest sehr kleines Stück in die proximale Richtung, d. h. entgegen die Ausschüttrichtung, bewegt wird, so dass der Kolben des Produktbehälters entlastet wird. Dabei kann die Ausschüttfeder wieder etwas gespannt werden. Vorzugsweise ist die Kupplung bereits geschlossen, wenn sich das Vortriebsglied in die proximale Richtung bewegt.

Das oben genannte Getriebe kann ein erstes Getriebeelement und ein zweites Getriebeelement aufweisen. Ein erstes Teil kann das erste Getriebeelement und ein zweites Teil das zweite Getriebeelement bilden, wobei das erste Getriebeelement das zweite Getriebeelement durch die Betätigung des Betätigungsglieds aneinander abgleiten, wodurch das Betätigen, insbesondere das Drücken des Betätigungsglieds bewirkt, dass sich das Rotationsglied relativ zu dem Gehäuse bereits dann, insbesondere in die erste Drehrichtung, dreht, wenn die Kupplung noch geschlossen ist.

In einer Ausführungsform kann das Gehäuse oder ein gehäusefestes Element das erste Teil und ein die Kupplung mitbildendes, zum Beispiel hülsenförmiges Verschiebeglied, insbesondere ein Lagerelement, welches zumindest axial verschiebbar in Bezug auf das Gehäuse oder das gehäusefeste Element ist, das zweite Teil sein. Das Verschiebeglied kann das gehäusefeste Element umgeben. Das erste Teil und das zweite Teil können so ineinandergreifen, dass eine durch Betätigen des Betätigungsglieds bewirkte Verschiebung des zweiten Teils eine Verdrehung des zweiten Teils relativ zu dem ersten Teil bewirkt. Beispielsweise kann eines aus erstem Teil und zweitem Teil ein Eingriffsglied aufweisen, wobei das andere aus erstem und zweitem Teil eine Führungsbahn aufweist, wobei eine Bewegung des Eingriffsglieds in der Führungsbahn eine Verdrehung des zweiten Teils relativ zu dem ersten Teil bewirkt. Zum Beispiel kann die Führungsbahn ein Gewinde oder ein Gewindeabschnitt sein. Beispielsweise kann die Führungsbahn einen proximalen und/oder einen distalen Führungsbahnabschnitt aufweisen, die über einen Übergangsabschnitt miteinander verbunden sind oder wobei sich ein Übergangsabschnitt an den proximalen oder distalen Führungsbahnabschnitt anschließt. Das Eingriffsglied kann, wenn es in der Führungsbahn, insbesondere in dem oder durch den Übergangsabschnitt bewegt wird, eine Verdrehung des zweiten Teils relativ zu dem ersten Teil bewirkt, insbesondere in die erste Drehrichtung, wenn das Betätigungsglied betätigt wird und sich das Eingriffsglied in eine erste Richtung in der Führungsbahn oder durch den oder in dem Übergangsabschnitt bewegt, und insbesondere in die zweite Drehrichtung, wenn das Betätigungsglied losgelassen wird und sich das Eingriffsglied in eine zweite Richtung, die der ersten Richtung entgegengesetzt ist, in der Führungsbahn oder durch den oder in dem Übergangsabschnitt bewegt. Zum Beispiel können der proximale Führungsbahnabschnitt und/oder der distale Führungsbahnabschnitt parallel zu der Längsachse sein, so dass das zweite Teil relativ zu dem ersten Teil drehfest und axial verschiebbar ist, wenn sich das Eingriffsglied in dem proximalen oder dem distalen Führungsbahnabschnitt befindet.

In einer weiteren Ausführungsform kann das die Kupplung mitbildende Rotationsglied das erste Teil und ein die Kupplung mitbildendes Verschiebeglied, insbesondere das Lagerelement, welches relativ zu dem Gehäuse oder einem gehäusefesten Element drehfest und entlang der Längsachse der Antriebsvorrichtung verschiebbar ist, das zweite Teil sein. Beispielsweise können das erste Teil und das zweite Teil während der Verschiebung des zweiten Teils, die durch das Betätigen oder das Loslassen des Betätigungsglieds verursacht wird, aneinander abgleiten, wodurch das erste Teil relativ zu dem Gehäuse gedreht wird, insbesondere in die erste Drehrichtung, wenn das Betätigungsglied betätigt wird, und/oder in die zweite Drehrichtung, wenn das Betätigungsglied losgelassen wird. Das erste Teil und das zweite Teil können ineinandergreifen. Beispielsweise kann das erste Teil Abragungen aufweisen, welche zu dem zweiten Teil weisen, wobei das zweite Teil Abragungen aufweisen kann, welche zu dem ersten Teil weisen. Die zueinander weisenden und ineinandergreifenden Abragungen können aneinander abgleiten, wenn das Betätigungsglied betätigt wird. Die Abragungen können zum Beispiel Teil der Kupplungsstruktur der genannten Kupplung sein, wobei die Kupplung erst gelöst ist, wenn die vorzugsweise zueinander weisenden Abragungen aneinander abgeglitten und aus dem Eingriff sind. Hierdurch kann sich das Rotationsglied relativ zu dem Gehäuse und dem zweiten Teil mittels der vorgespannten Ausschüttfeder in die erste Drehrichtung drehen.

In noch weiteren Ausführungen kann das Rotationsglied eine Verzahnung oder Vielzahl über seinen Umfang angeordnete, z. B. sägezahnförmige, Zähne aufweisen, in die eine Rastnocke, die von einer über den Umfang des Rotationsglieds angeordneten Zwischenhülse, gebildet ist, federnd eingreift, wobei die Zwischenhülse das erste Teil ist und eine Kupplungsstruktur der Kupplung bildet und ein Verschiebeglied, insbesondere ein Lagerelement, welches drehfest und axial verschiebbar in Bezug auf das Gehäuse oder ein gehäusefestes Element ist, das zweite Teil ist, wobei das bevorzugt hülsenförmige Verschiebeglied die Zwischenhülse über ihren Umfang umgibt. Die Zwischenhülse kann konzentrisch zwischen dem Rotationsglied und dem Verschiebeglied angeordnet sein. Zum Beispiel kann eines aus Verschiebeglied und Zwischenhülse eine Abragung aufweisen, welche in eine Führungsbahn des anderen aus Verschiebeglied und Zwischenhülse eingreift. Zum Beispiel kann die Führungsbahn ein Gewinde oder ein Gewindeabschnitt sein. Beispielsweise kann die Führungsbahn einen proximalen und/oder einen distalen Führungsbahnabschnitt aufweisen, die über einen Übergangsabschnitt miteinander verbunden sind oder wobei sich ein Übergangsabschnitt an den proximalen oder distalen Führungsbahnabschnitt anschließt. Die Abragung bewirkt, wenn sie in der Führungsbahn, insbesondere in dem Übergangsabschnitt, bewegt wird, eine Verdrehung der Zwischenhülse und insbesondere des Rotationsglieds relativ zu dem Verschiebeglied, insbesondere in die erste Drehrichtung, wenn das Betätigungsglied betätigt wird und sich die Abragung in eine erste Richtung in der Führungsbahn, insbesondere durch den oder in dem Übergangsabschnitt bewegt, und insbesondere in die zweite Drehrichtung, wenn das Betätigungsglied losgelassen wird und sich die Abragung in eine zweite Richtung, die der ersten Richtung entgegengesetzt ist, in der Führungsbahn, insbesondere durch den oder in dem Übergangsabschnitt bewegt. Zum Beispiel können der proximale Führungsbahnabschnitt und/oder der distale Führungsbahnabschnitt parallel zu der Längsachse sein, so dass die Zwischenhülse relativ zu dem Verschiebeglied drehfest und axial verschiebbar ist, wenn sich die Abragung in dem proximalen oder dem distalen Führungsbahnabschnitt befindet. Vorteilhaft bewirken die Führungsbahn und die in die Führungsbahn eingreifende Abragung, dass sich die Zwischenhülse in die erste Drehrichtung dreht, wenn das Betätigungsglied betätigt wird, d. h. aus seiner unbetätigten Position in seine betätigte Position verschoben wird.

Der Eingriff der Rastnocke in die über den Umfang angeordneten Zähne ist so, dass eine Drehung der Zwischenhülse relativ zu dem Rotationsglied in die erste Drehrichtung verhindert wird und eine Drehung des Rotationsglieds relativ zu der Zwischenhülse in die erste Drehrichtung möglich ist. Die Betätigung des Betätigungsglieds bewirkt eine Drehung der Zwischenhülse in die erste Drehrichtung relativ zu dem Gehäuse, wobei die Zwischenhülse das Rotationsglied mitnimmt, d. h. mitdreht. Wenn das Betätigungsglied in seiner betätigten Position ist, kann die Antriebsfeder das Rotationsglied relativ zu der Zwischenhülse in die erste Drehrichtung drehen, da die Kupplung geöffnet ist. Beispielsweise kann das Kupplungsglied, insbesondere das Lagerelement oder dessen Innenfläche, wie z. B. der Innenumfang, die Rastnocke daran hindern, aus der Verzahnung des Rotationsglieds heraus bewegt zu werden, wenn das Betätigungsglied unbetätigt oder nicht vollständig betätigt, d. h. nicht in seiner betätigten Position ist. Vorzugweise gibt das Verschiebeglied, insbesondere das Lagerelement eine Bewegung der Rastnocke aus der Verzahnung frei, wenn das Betätigungsglied vollständig betätigt, d. h. in seiner betätigten Position ist. Das Verschiebeglied kann hierfür eine Ausnehmung aufweisen, die mittels Betätigen des Betätigungsglieds über die Rastnocke geschoben wird, so dass die Rastnocke sich aus dem Eingriff mit der Verzahnung bewegen kann. Alternativ oder zusätzlich kann die Innenfläche des Verschiebeglieds, insbesondere des Lagerelements, aus der Position, in der sie die Rastnocke in dem Eingriff mit der Verzahnung hält, bewegt werden, so dass sich die Rastnocke aus dem Eingriff mit der Verzahnung bewegen kann. Vorzugsweise bewegt sich die Rastnocke mit einer Bewegung in etwa radial nach außen, d. h. von der Längsachse weg, aus dem Eingriff mit der Verzahnung und mit einer Bewegung in etwa radial nach innen, d. h. zu der Längsachse hin, in die Verzahnung.

Vorzugsweise ist die Ausschüttfeder kinematisch zwischen dem Kolben des Produktbehälters und dem Rotationsglied angeordnet. Hierdurch kann vermieden werden, dass die von der Ausschüttfeder bereitgestellte Ausschüttenergie zum großen Teil über das Rotationsglied laufen muss, wie es z. B. der Fall wäre, wenn das Rotationsglied kinematisch zwischen der Ausschüttfeder und dem Kolben angeordnet wäre. Dadurch lässt sich das Rotationsglied einfacher ausgestalten. Insbesondere kann somit erreicht werden, dass die mindestens eine Ausschüttfeder das Vortriebsglied antreibt und das Vortriebsglied das Rotationsglied antreibt. Insbesondere kann das Vortriebsglied kinematisch zwischen der Ausschüttfeder und dem Rotationsglied angeordnet sein.

Insbesondere kann der Drehwinkel des Rotationsglieds proportional zu dem Ausschütthub des Kolbens oder des Vortriebsglieds sein. Dies lässt sich durch die selektive Sperrung oder Freigabe des Rotationsglieds erreichen.

Vorteilhaft kann das Rotationsglied in einem Eingriff, insbesondere einem Gewindeeingriff mit dem Vortriebsglied sein. Durch die Gewindesteigung dieses Gewindeeingriffs wird erreicht, dass z. B. bei einer vollständigen Umdrehung des Rotationsglieds relativ zu dem Gehäuse das Vortriebsglied von der Ausschüttfeder um einen Hub verschiebbar ist, welcher der Gewindesteigung entspricht.

Z. B. können das Rotationsglied eine Gewindestange und das Vortriebsglied eine Gewindemutter aufweisen oder sein, wobei das Gewinde der Gewindemutter in das Gewinde der Gewindestange eingreift.

In einem alternativen Beispiel können das Rotationsglied eine Gewindemutter und das Vortriebsglied eine Gewindestange aufweisen oder sein, wobei das Gewinde der Gewindemutter in das Gewinde der Gewindestange eingreift.

Vorzugsweise ist das Rotationsglied axialfest in Bezug auf das Gehäuse oder kann sich zumindest in eine, vorzugsweise in distale Richtung axialfest an dem Gehäuse oder einem gehäusefesten Element, wie z. B. dem Widerlager abstützen.

Es ist vorteilhaft, dass das Rotationsglied während des Einstellens einer Dosis, d. h. im unbetätigten Zustand, insbesondere mittels der Kupplung, drehfest mit dem Gehäuse verbunden ist und während der Betätigung der Vorrichtung zum Ausschütten der Produktdosis relativ zu dem Gehäuse gedreht wird oder drehbar ist.

In Ausführungen, bei denen eine Produktdosis einstellbar ist, weist die Antriebs- und Dosiervorrichtung ein Dosisanzeigeelement, insbesondere eine Dosisanzeigetrommel auf. Das Dosisanzeigeelement kann zum Beispiel in einem Gewindeeingriff mit dem Gehäuse oder einem gehäusefesten Element sein, wodurch das Dosisanzeigeelement an dem Gehäuse durch Drehen eines Dosiseinstellglieds entlang schraubbar ist. Alternativ kann das Dosisanzeigeelement in einem Gewindeeingriff mit dem Verschiebeglied, insbesondere dem Lagerelement sein.

Das Dosisanzeigeelement, insbesondere die Dosisanzeigetrommel kann während des Einstellens einer Dosis, d. h. im unbetätigten Zustand der Antriebs- und Dosiervorrichtung oder des Betätigungsglieds relativ zu dem Rotationsglied drehbar sein. Vorzugsweise ist das Dosisanzeigeelement während der Betätigung der Vorrichtung zum Ausschütten der Produktdosis relativ zu dem Rotationsglied drehfest und z. B. axial bewegbar, oder drehfest mit dem Rotationsglied verbunden, insbesondere mit der beschriebenen Kupplung oder einer weiteren Kupplung.

Vorteilhaft wird bewirkt, dass bei der Dosisausschüttung, d. h. wenn sich das Betätigungsglied in seiner betätigten Position befindet, die Ausschüttfeder das Dosisanzeigeelement in seine Nulldosisposition zurückschraubt, insbesondere über das Rotationsglied und vorzugsweise über ein Kupplungsglied, welches vorzugsweise drehfest aber axial verschiebbar in Bezug auf das Dosisanzeigeelement angeordnet ist. Insbesondere können das Kupplungsglied und das Dosisanzeigeelement in einem drehfesten Eingriff sein, der eine Axialbewegung zwischen Dosisanzeigeelement und Kupplungsglied zulässt. Dieser Eingriff kann z. B. mittels einer Längsführung bewirkt werden. Bevorzugt ist das Kupplungsglied axialfest aber drehbar mit dem Verschiebeglied, insbesondere dem Lagerelement, verbunden.

Beispielsweise kann die Antriebs- und Dosiervorrichtung eine erste Kupplungsstruktur, die in Bezug auf das Gehäuse drehfest ist, aufweisen. Das Rotationsglied kann eine zweite Kupplungsstruktur aufweisen oder bilden, die im Kupplungseingriff mit der ersten Kupplungsstruktur bewirkt, dass das Rotationsglied drehfest in Bezug auf das Gehäuse ist. Die erste Kupplungsstruktur kann z. B. von dem Gehäuse oder einem drehfest in Bezug auf das Gehäuse angeordneten aber axial verschiebbaren Verschiebeglied, wie z. B. dem Lagerelement gebildet sein.

Das Kupplungsglied kann eine dritte Kupplungsstruktur aufweisen, die in einem Eingriff mit der ersten Kupplungsstruktur oder einer weiteren, vierten Kupplungsstruktur des Rotationsglieds bewirkt, dass das Dosisanzeigeelement drehfest mit dem Rotationsglied verbunden ist. Wenn das Betätigungsglied in seiner unbetätigten Position ist, sind das Rotationsglied und das Gehäuse im Bezug zueinander drehfest, insbesondere die erste und die zweite Kupplungsstruktur in einem Eingriff, wobei die dritte und zweite oder optional die dritte und vierte Kupplungsstruktur eingriffsfrei sind. Wenn das Betätigungsglied in seiner betätigten Position ist, sind die dritte und zweite, optional die dritte und vierte Kupplungsstruktur im Eingriff, wobei die erste und zweite Kupplungsstruktur vorzugsweise voneinander gelöst sind.

Besonders vorteilhaft ist es, wenn das Dosisanzeigeelement bereits drehfest mit dem Rotationsglied gekoppelt ist und das Rotationsglied noch drehfest mit dem Gehäuse verbunden ist, während das Betätigungsglied für die Betätigung in Bezug auf das Gehäuse verschoben wird. Hierdurch wird sichergestellt, dass das Dosisanzeigeelement zuerst sicher mit dem Rotationsglied gekoppelt ist, wenn das Rotationsglied für eine Drehung relativ zu dem Gehäuse freigegeben ist. Mit anderen Worten gibt es zwischen der betätigten und unbetätigten Position des Betätigungsglieds eine Zwischenposition, in der das Rotationsglied sowohl drehfest mit dem Gehäuse als auch drehfest mit dem Dosisanzeigeelement gekoppelt ist. Insbesondere können die erste und die zweite und die dritte und die zweite, optional die dritte und die vierte Kupplungsstruktur gleichzeitig in einem Eingriff sein, nämlich dann, wenn das Betätigungsglied seine Zwischenposition einnimmt.

In allgemein bevorzugten Ausführungen kann das Dosisanzeigeelement einen Anschlag, wie z. B. einen Nulldosisanschlag aufweisen, der von einem Gegenanschlag, insbesondere einem Nulldosisgegenanschlag wegbewegt wird, wenn eine Dosiserhöhung vorgenommen wird, und der zu dem Gegenanschlag hinbewegt wird, wenn eine Dosisverringerung vorgenommen wird, oder wenn die Vorrichtung für die Ausschüttung der eingestellten Produktdosis betätigt wird.

Insbesondere kann das Dosisanzeigeelement während des Einstellens der Produktdosis, d. h. bei Dosiserhöhung und Dosisverringerung, von dem Rotationsglied zumindest rotatorisch entkoppelt sein und bei der Betätigung der Vorrichtung zum Ausschütten der Produktdosis so mit dem Rotationsglied gekoppelt sein, dass eine Drehung des Rotationsglieds bewirkt, dass das Dosisanzeigeelement zu dem Gegenanschlag, d. h. der Nulldosisanschlag zu dem Nulldosisgegenanschlag hin bewegt wird. Sind der Nulldosisanschlag und der Nulldosisgegenanschlag in einem Anschlag oder einem Kontakt, wird hierdurch und insbesondere über die Kupplung, eine Drehung des Rotationsglieds und somit ein weiterer Vorschub des Vortriebsglieds relativ zu dem Gehäuse verhindert.

Zwischen dem Dosiseinstellglied und dem Dosisanzeigeelement kann eine Dosierkupplung vorgesehen sein, welche das Dosiseinstellglied mit dem Dosisanzeigeelement drehfest koppelt, wenn die Antriebs- und Dosiervorrichtung oder das Betätigungsglied unbetätigt ist, und rotatorisch entkoppelt, wenn die Antriebs- und Dosiervorrichtung oder das Betätigungsglied betätigt ist. Mit anderen Worten ausgedrückt, sind das Dosisanzeigeelement und das Dosierglied über die Dosierkupplung drehfest verbunden, wenn das Betätigungsglied unbetätigt ist und ist das Dosisanzeigeelement relativ zu dem Dosiseinstellglied drehbar, wenn das Betätigungsglied betätigt ist. Durch Betätigung des Betätigungsglieds wird die Dosierkupplung geöffnet.

In vorteilhaften Weiterbildungen kann die Antriebs- und Dosiervorrichtung einen Mechanismus zum Verhindern des Einstellens einer Dosis, welche die Menge eines Medikaments in dem Produktbehälter übersteigt, aufweisen. Insbesondere kann dieser Mechanismus die Drehung des Dosierglieds in eine Richtung, welche eine Dosiserhöhung bewirken würde blockieren, insbesondere auch wenn der Maximaldosisanschlag des Dosisanzeigeelements und der Maximaldosisgegenanschlag noch nicht in einem Eingriff sind oder wenn zum Beispiel in einer Zeigeeinrichtung eine Dosis angezeigt wird, die kleiner ist als die maximal einstellbare Produktdosis. Der Mechanismus verhindert somit, dass eine Dosis eingestellt werden kann, welche die Restmenge des in dem Produktbehälter enthaltenen Produkts übersteigt, wodurch die Gefahr einer Fehlanwendung der Antriebs- und Dosiervorrichtung verringert wird. Der Mechanismus kann z. B. einen Begrenzer aufweisen, der zwischen zwei Teilen angebracht ist, von denen sich eines relativ zu dem anderen während der Dosiseinstellung dreht und bei der Betätigung, d. h. der Dosisausschüttung nicht dreht. Z. B. kann der Begrenzer zwischen dem Dosiseinstellglied, das insbesondere als Dosiseinstellknopf oder Dosiseinstellhülse ausgestaltet sein kann, und dem Gehäuse oder einem gehäusefesten Element angeordnet sein. Der Begrenzer, das Dosiseinstellglied und das Gehäuse können so miteinander gekoppelt sein, dass eine relative Drehung, insbesondere während der Dosiseinstellung, zwischen dem Dosiseinstellglied und dem Gehäuse bewirkt, dass sich der Begrenzer zu einer Stoppposition hinbewegt, in welcher der Begrenzer das Einstellen einer Dosis verhindert, die die Menge eines Produkts in dem Produktbehälter übersteigt. Beispiele für entsprechend geeignete Begrenzer werden in der WO 2010/149209 A1 oder in der WO 01/19434 A1, insbesondere in deren Figur 3 offenbart. Beispielsweise kann der Begrenzer ein Innengewinde aufweisen, welches in dem Eingriff mit einem Außengewinde des Gehäuses ist. Insbesondere kann der Begrenzer an seiner Außenseite eine Längsführung aufweisen, mit der er in einem Eingriff mit dem Dosiseinstellglied ist, so dass das Dosiseinstellglied relativ zu dem Begrenzer drehfest ist. Alternativ kann das Gehäuse die Längsführung für den Begrenzer aufweisen, so dass der Begrenzer relativ zu dem Gehäuse drehfest ist und kann der Begrenzer ein Gewinde, insbesondere Außengewinde aufweisen, welches in ein Gewinde, insbesondere Innengewinde des Dosiseinstellglieds eingreift.

Die Stoppposition wird durch einen Anschlag für den Begrenzer definiert, wobei der Anschlag von dem Gehäuse oder dem Dosiseinstellglied oder einem zumindest axial oder in Umfangsrichtung gehäusefesten Mittel gebildet werden kann. Sind der Begrenzer und der Anschlag in einem Kontakt, ist eine Drehung des Dosiseinstellglieds in eine Drehrichtung, welche eine Erhöhung der Dosis bewirken würde, nicht mehr möglich oder blockiert.

In allgemein bevorzugten Weiterbildungen kann die Antriebs- und Dosiervorrichtung optional mindestens einen Signalerzeugungsmechanismus aufweisen, der angepasst ist, während der Dosiseinstellung oder/und der Produktausschüttung ein akustisches und/oder taktiles Signal, insbesondere mechanisch, zu erzeugen. Ein solches Signal kann insbesondere als Klicksignal wahrgenommen werden. Zum Beispiel kann ein (erster) Signalerzeugungsmechanismus vorgesehen sein, der das Signal während der Dosiseinstellung erzeugt und optional als Dosiseinstellungssignalerzeugungsmechanismus bezeichnet werden kann. Alternativ oder zusätzlich kann ein (zweiter) Signalerzeugungsmechanismus vorgesehen sein, der das Signal während der Produktausschüttung erzeugt und optional als Produktausschüttungssignalerzeugungsmechanismus bezeichnet werden kann. Alternativ kann ein (gemeinsamer) Signalerzeugungsmechanismus vorgesehen sein, der das Signal während der Dosiseinstellung und während der Produktausschüttung erzeugt.

Der Signalerzeugungsmechanismus kann allgemein zwischen zwei Teilen angeordnet sein, die sich bei der Dosiseinstellung oder/und der Produktausschüttung relativ zueinander bewegen, insbesondere drehen. Eines der Teile kann ein z. B. federnd angeordnetes Rastglied aufweisen, das in eine z. B. über den Umfang angeordnete Verzahnung des anderen der zwei Teile eingreift. Wird ein Teil relativ zu dem anderen verdreht, kann das Rastglied über die Verzahnung gleiten und dabei das Signal erzeugen. Die Verzahnung kann von einem Innenumfang oder einem Außenumfang oder einer Stirnfläche des Teils gebildet sein. Zum Beispiel kann der Signalerzeugungsmechanismus für die Produktausschüttung von der Nocke der Zwischenhülse, die weiter oben im Zusammenhang mit einer Ausführungsform beschrieben wird, gebildet werden.

Der Signalerzeugungsmechanismus kann insbesondere zwischen Kupplungsglied und Verschiebeglied bzw. Lagerelement gebildet sein. Vorzugsweise drehen sich das Kupplungsglied und das Verschiebeglied bzw. das Lagerelement während der Dosiseinstellung und der Produktausschüttung relativ zueinander, wodurch ein Signalerzeugungsmechanismus gebildet wird, der das Signal während der Dosiseinstellung und der Produktausschüttung erzeugt.

Der Signalerzeugungsmechanismus kann insbesondere zwischen Verschiebeglied bzw. Lagerelement und Rotationsglied gebildet sein, wobei das für das Verschiebeglied bzw. Lagerelement ausgeführte, zumindest in diesem Zusammenhang, auch für eine hierin beschriebene Schalthülse gilt. Vorzugsweise drehen sich das Verschiebeglied bzw. das Lagerelement und das Rotationsglied, während, insbesondere nur während der Produktausschüttung relativ zueinander, wodurch ein Signalerzeugungsmechanismus gebildet wird, der das Signal während der Produktausschüttung erzeugt.

Der Signalerzeugungsmechanismus kann insbesondere zwischen Kupplungsglied und Rotationsglied gebildet sein. Vorzugsweise drehen sich das Kupplungsglied und das Rotationsglied, während, insbesondere nur während der Dosiseinstellung relativ zueinander, wodurch ein Signalerzeugungsmechanismus gebildet wird, der das Signal während der Dosiseinstellung erzeugt.

Über den Umfang des Dosisanzeigeelements kann eine Dosisskala angeordnet sein. Das Dosisanzeigeelement kann z. B. im Querschnitt ringförmig sein. Das Dosisanzeigeelement kann z. B. eine Dosisanzeigetrommel oder ein Dosisanzeigering sein. Die Dosisskala kann sich über den Umfang des Dosisanzeigeelements, vorzugsweise wendelförmig, erstrecken. Die Dosisskala umfasst vorzugsweise eine Vielzahl von Werten, die aneinandergereiht angeordnet sind und die Dosisskala ergeben. Vorzugsweise handelt es sich um Zahlenwerte, welche die gewünschte Produktdosis in internationalen Einheiten (I.U.) angeben.

Alternativ kann die Dosisskala ohne Steigung über den Umfang des Dosisanzeigeelements, wie z. B. des Dosisanzeigerings, angeordnet sein, wobei sich die Skalenwerte dann nach einer Umdrehung des Dosisanzeigeelements wiederholen. Bei einer Dosisskala mit Steigung, d. h. einer wendelförmigen Dosisskala kann das Dosisanzeigeelement, insbesondere die Dosisanzeigetrommel, mit mehr als einer Umdrehung gedreht werden, ohne dass sich die Skalenwerte wiederholen, wodurch vorteilhaft die Skalenwerte größer oder mehr Skalenwerte dargestellt werden können.

Die Antriebs- und Dosiervorrichtung umfasst ferner eine Zeigeeinrichtung, wobei das Dosisanzeigeelement zur Einstellung der zu verabreichenden Dosis relativ zu der Zeigeeinrichtung und insbesondere um eine Drehachse, die vorzugsweise der Längsachse der Antriebs- und Dosiervorrichtung oder/und des Dosisanzeigeelements entspricht, drehbar ist. Hierbei kann es sich um eine reine Drehbewegung, d. h. eine Drehbewegung ohne überlagerte Axialbewegung handeln. Vorzugsweise ist der Drehbewegung eine Axialbewegung überlagert, wodurch das Dosisanzeigeelement zur Einstellung der zu verabreichenden Dosis relativ zu der Zeigeeinrichtung schraubbar ist. Ein schraubbares Dosisanzeigeelement lässt sich vorteilhaft mit einer wendelförmigen Dosisskala kombinieren, wobei die Schraubbewegung und die Dosisskala vorteilhafterweise die gleiche Steigung aufweisen. Ein ohne Axialbewegung drehbares Dosisanzeigeelement lässt sich vorteilhaft mit einer steigungsfreien Dosisskala kombinieren.

Mittels der Zeigeeinrichtung, die bevorzugt an dem Gehäuse gebildet wird, ist ein Wert der Dosisskala ablesbar, welcher der eingestellten Dosis entspricht. Die Zeigeeinrichtung kann z. B. ein Fenster sein, das durch einen Durchbruch im Gehäuse oder durch einen transparenten Einsatz gebildet sein kann. Alternativ oder optional kann die Zeigeeinrichtung ein Pfeil sein oder einen Pfeil aufweisen, der z. B. zusätzlich zu dem Fenster den Wert der Dosisskala markiert, welcher der eingestellten Dosis entspricht. Dies ist z. B. dann von Vorteil, wenn in dem Fenster noch ein anderer Wert zumindest teilweise erscheint, um eine eindeutige Dosisauswahl sicherzustellen. Der Zeiger kann z. B. ein Vorsprung oder ein Aufdruck oder eine Kerbe oder dergleichen sein.

Das Dosierglied ist vorzugsweise vom Verwender (Patient, Arzt, medizinisches Hilfspersonal) der Antriebs- und Dosiervorrichtung greibar und bildet vorzugsweise eine äußere, insbesondere von außen zugängliche Oberfläche der Antriebs- und Dosiervorrichtung. Zur Einstellung der auszuschüttenden oder zu verabreichenden Dosis wird das Dosierglied vorzugsweise vom Verwender ergriffen und relativ zu dem Gehäuse und insbesondere der Zeigeeinrichtung um eine Drehachse, die vorzugsweise der Längsachse der z. B. länglich ausgestalteten Antriebs- und Dosiervorrichtung entspricht, verdreht. Das Dosierglied ist vorzugsweise axialfest mit dem Gehäuse verbunden, insbesondere entlang einer Längsachse des Gehäuses verschiebefest, wodurch vorteilhaft die intuitive Handhabung der Vorrichtung durch den Verwender erleichtert wird, da er lediglich eine Drehbewegung des Dosierglieds zur Dosiseinstellung ausführen braucht.

Insbesondere kann das Dosisanzeigeelement zumindest während der Dosiseinstellung verdrehfest, aber z. B. axial verschiebbar mit dem Dosierglied verbunden oder gekoppelt sein. Für die intuitive Bedienung ist es vorteilhaft, wenn das Dosisanzeigeelement bei Drehung des Dosierglieds um einen Drehwinkel ebenfalls um diesen Drehwinkel verdreht wird.

Das vorzugsweise als Betätigungsknopf ausgestaltete Betätigungsglied kann eine äußere Oberfläche der Antriebsvorrichtung bilden und/oder von außen zugänglich sein. Das Betätigungsglied kann am proximalen, insbesondere hinteren Ende der Antriebsvorrichtung gebildet sein oder dieses Ende bilden. Das Betätigungsglied lässt sich auf diese Weise vorteilhaft mit dem Daumen der Hand, die das Gehäuse umgreift betätigen, insbesondere drücken. Durch Loslassen des Betätigungsglieds kann die Betätigung beendet werden.

Das Betätigungsglied kann vorteilhaft gegen die Kraft einer Feder, insbesondere einer Rücksetz- oder Kupplungsfeder verschiebbar, insbesondere betätigbar sein, wodurch diese Feder gespannt wird. Durch Loslassen kann diese Feder das Betätigungsglied zurücksetzen, insbesondere relativ zu dem Dosierglied, insbesondere in proximale Richtung oder aus der Antriebsvorrichtung heraus, verschieben.

Die Antriebs- und Dosiervorrichtung kann ferner ein Lagerelement, mit dem das Dosisanzeigeelement in einem Eingriff ist, umfassen. Dieser Eingriff bewirkt vorteilhaft die Dreh- oder Schraubbewegung des Dosisanzeigeelements relativ zu der Zeigeeinrichtung. Z. B. kann der Eingriff zwischen Dosisanzeigeelement und Verschiebeglied, insbesondere Lagerelement, ein Gewindeeingriff sein. Insbesondere kann das Lagerelement ein Außengewinde und das Dosisanzeigeelement ein Innengewinde aufweisen, wobei diese Gewinde ineinander greifen und dadurch bewirken, dass das Dosisanzeigeelement relativ zu dem Lagerelement schraubbar ist.

Insbesondere ist das Dosisanzeigeelement zwischen einer Maximaldosisposition und einer Nulldosisposition hin und her dreh- oder schraubbar. In der Nulldosisposition kann vorteilhaft die Dosis oder Ziffer "0" in der Zeigeeinrichtung ablesbar sein. In der Maximaldosisposition kann vorteilhaft die maximal mit der Antriebs- und Dosiervorrichtung ausschüttbare Produktdosis ablesbar sein.

In der Nulldosisposition kann das Dosisanzeigeelement gegen die Drehung in eine Drehrichtung gesperrt sein, nämlich in die Drehrichtung, die bewirken würde, dass eine Dosis kleiner als null eingestellt wird. In der Nulldosisposition kann das Dosisanzeigeelement vorzugsweise nur in die Drehrichtung bewegt werden, die eine Erhöhung der Dosis bewirkt. In der Maximaldosisposition ist das Dosisanzeigeelement vorzugsweise gegen die Drehung in eine Drehrichtung, nämlich in die Drehrichtung, die die Einstellung einer Dosis über die maximal einstellbare Dosis hinaus bewirken würde, blockiert. Das Dosisanzeigeelement kann in der Maximaldosisposition insbesondere nur in die Drehrichtung gedreht werden, die eine Verringerung der Produktdosis bewirkt.

Das Dosisanzeigeelement kann z. B. einen Anschlag aufweisen, der in der Nulldosisposition an einen Gegenanschlag anschlägt und somit die Drehung in eine Drehrichtung verhindert. Der gleiche oder ein zusätzlicher Anschlag des Dosisanzeigeelements kann die Drehung des Dosisanzeigeelements über die Maximaldosis hinaus verhindern. Insbesondere kann hierfür ein weiterer Gegenanschlag, nämlich ein Maximaldosisgegenanschlag vorgesehen sein. Dementsprechend kann der andere Gegenanschlag als Nulldosisgegenanschlag bezeichnet werden. Das Dosisanzeigeelement kann demnach einen Nulldosisanschlag für den Nulldosisgegenanschlag und einen Maximaldosisanschlag für den Maximaldosisgegenanschlag aufweisen. Vorzugsweise wirken der Anschlag oder die Anschläge in Umfangsrichtung und/oder in Axialrichtung.

Das Lagerelement kann zusammen mit dem Dosisanzeigeelement relativ zu dem Gehäuse und entlang der Drehachse, insbesondere in distale Richtung verschiebbar sein. Alternativ kann das Dosisanzeigeelement ein Gewinde aufweisen, welches im Eingriff mit dem Gehäuse ist. Dadurch lässt sich das Dosisanzeigeelement zwar relativ zu dem Gehäuse hin und her schrauben, aber nicht unabhängig von der Schraubbewegung mit einer insbesondere reinen Axialbewegung verschieben.

Vorzugsweise ist das Betätigungsglied so mit dem Lagerelement gekoppelt, dass eine Verschiebung des Betätigungsglieds relativ zu dem Gehäuse und/oder dem Dosiseinstellglied eine Verschiebung des Lagerelements relativ zu dem Gehäuse und/oder dem Dosiseinstellglied insbesondere entlang der Längsachse der Antriebs- oder der Antriebs- und Dosiervorrichtung bewirkt.

Dadurch dass das Dosisanzeigeelement in einem Eingriff mit dem Lagerelement ist und sich das Lagerelement relativ zu dem Gehäuse und entlang der Drehachse verschieben lässt, lässt sich auch das Dosisanzeigeelement relativ zu dem Gehäuse und entlang der Drehachse unabhängig von der Dreh- oder Schraubbewegung, welche das Dosisanzeigeelement bei der Dosiseinstellung ausführt, verschieben. Die Antriebs- und Dosiervorrichtung lässt sich im Grunde aber auch vorteilhaft mit dem alternativen Dosisanzeigeelement kombinieren, das in dem Gewindeeingriff mit dem Gehäuse oder einem gehäusefesten Element ist. In dieser Alternative kann das Lagerelement von dem Gehäuse gebildet sein oder Teil des Gehäuses sein, wobei das Lagerelement dann z. B. dreh- und axialfest in Bezug auf das übrige Gehäuse sein kann. Das Verschiebeglied und das Lagerelement sind dann zum Beispiel separate Teile.

Vorteilhaft kann an der Zeigeeinrichtung und/oder an dem Dosisanzeigeelement abgelesen werden, dass das Lagerelement zusammen mit dem Dosisanzeigeelement verschoben wurde. Hierdurch lässt sich durch den Verwender kontrollieren, in welchem Betriebszustand sich die Antriebs- und Dosiervorrichtung befindet, d. h. ob die Antriebs- und Dosiervorrichtung insbesondere das Betätigungsglied für eine Ausschüttung betätigt oder unbetätigt ist.

In einer bevorzugten Variante kann das Betätigungsglied oder/und das Lagerelement zusammen mit dem Dosisanzeigeelement relativ zu der Zeigeeinrichtung, dem Gehäuse und entlang der Drehachse verschiebbar sein. Im Bereich der Zeigeeinrichtung, insbesondere in dem Fenster der Zeigeeinrichtung kann eine von der Dosisskala verschiedene Markierung erscheinen, wenn das Lagerelement verschoben ist. Die Markierung ist vorzugsweise an dem Dosisanzeigeelement angeordnet. Wenn das Lagerelement unverschoben ist, insbesondere die Antriebs- und Dosiervorrichtung für die Produktausschüttung unbetätigt ist, kann die Markierung außerhalb der Zeigeeinrichtung angeordnet sein, wie z. B. von einem Gehäuse oder einem anderen Element verdeckt sein. Wird das Lagerelement verschoben, insbesondere die Antriebs- und Dosiervorrichtung für die Produktausschüttung betätigt, kann die Markierung aus dem abgedeckten Bereich hervortreten, so dass sie insbesondere an oder in der Zeigeeinrichtung erscheint oder ablesbar ist. Wird die Betätigung der Antriebs- und Dosiervorrichtung unterbrochen oder beendet, kann das Lagerelement in die Ursprungsposition zurückkehren, wodurch die Markierung vorzugsweise aus dem Bereich der Zeigeeinrichtung entfernt und insbesondere verdeckt wird.

In einer alternativen Variante kann das Betätigungsglied oder/und das Lagerelement zusammen mit dem Dosisanzeigeelement und der Zeigeeinrichtung relativ zu dem Gehäuse und entlang der Drehachse verschiebbar sein. Die Zeigeeinrichtung kann z. B. eine Blende sein oder zumindest die Funktion einer Blende erfüllen. Z. B. kann die Zeigeeinrichtung zumindest axialfest, vorzugsweise auch drehfest mit dem Lagerelement verbunden sein. Im Grunde kann das Lagerelement die Zeigeeinrichtung bilden. Selbstverständlich ist es aber auch möglich, dass die Zeigeeinrichtung ein von dem Lagerelement separates Teil ist. Die Zeigeeinrichtung kann z. B. hülsenförmig sein.

In dieser Variante kann die Verschiebung des Lagerelements bewirken, dass im Bereich der Zeigeeinrichtung eine von der Dosisskala verschiedene Markierung erscheint, die an oder auf der Zeigeeinrichtung angeordnet oder gebildet ist. Beispielsweise kann die Zeigeeinrichtung innerhalb des Gehäuses angeordnet sein. Die Markierung der Zeigeeinrichtung kann in unbetätigtem Zustand der Antriebs- und Dosiervorrichtung von dem Gehäuse oder einem anderem Element verdeckt sein. Wird die Antriebs- und Dosiervorrichtung, insbesondere das Betätigungsglied, betätigt, so dass das Dosisanzeigeelement zusammen mit der Zeigeeinrichtung verschoben wird, kann die Markierung aus seiner Abdeckung hervortreten, so dass die Markierung erblickbar oder ablesbar ist. Wird die Betätigung unterbrochen oder beendet, kann das Dosisanzeigeelement zusammen mit der Zeigeeinrichtung und dem Lagerelement in seine Ausgangsposition zurückverschoben werden, so dass die Markierung wieder unter der Abdeckung angeordnet ist.

Allgemein bevorzugt kann bei der Betätigung der Antriebsvorrichtung für eine Produktausschüttung eine Feder, insbesondere eine Kupplungs- oder Rückstellfeder gespannt werden. Beispielsweise kann das Lagerelement bei der Betätigung gegen die Kraft einer insbesondere solchen Feder verschoben werden, insbesondere aus einer unbetätigten Position in eine betätigte Position. Die Feder kann z. B. eine Schrauben- oder Wendelfeder sein, die als Druckfeder wirkt. Diese Feder bewirkt ferner, dass beim Unterbrechen oder Beenden der Betätigung das Lagerelement in seine Ausgangsposition oder unbetätigte Position zurückgesetzt wird. Insbesondere wird das Lagerelement bei der Betätigung in distale Richtung verschoben. Mittels der Feder wird das Lagerelement in proximale Richtung zurück verschoben, wenn die Betätigung unterbrochen oder beendet wird.

Die Betätigung des Betätigungsglieds bewirkt insbesondere, dass das Lagerelement zusammen mit dem Dosisanzeigeelement relativ zu dem Gehäuse und entlang der Drehachse verschoben wird.

Das Betätigungsglied ist vorzugsweise mit dem Verschiebeglied, insbesondere dem Lagerelement so verbunden, dass es das Verschiebeglied bei Betätigung verschiebt, insbesondere über ein Kupplungsglied, das z. B. axialfest und drehbar mit dem Verschiebeglied verbunden sein kann.

In allgemein bevorzugten Ausführungen kann die Betätigung des Betätigungsglieds bewirken, dass das Dosisanzeigeelement relativ zu oder an dem Lagerelement oder dem Gehäuse gedreht, insbesondere geschraubt wird, insbesondere in eine Richtung, dass die sich bei der Drehbewegung an der Zeigeeinrichtung vorbei bewegenden Werte der Dosisskala zurückzählen. Vorzugsweise stehen der Drehwinkel des Dosisanzeigeelements und der Ausschütthub des Vortriebsglieds in einem proportionalen Zusammenhang, insbesondere zu jedem Zeitpunkt während der Dosisausschüttung. Hierdurch lässt sich eine Echtzeitanzeige verwirklichen, die bei der Dosisausschüttung zurückzählt, bis sie schließlich beim Wert 0 angelangt, wobei die Ausschüttung dieser Dosis dann beendet ist. Wird die Betätigung für die Ausschüttung während des Zurückdrehens des Dosisanzeigeelements unterbrochen, zeigt das Dosisanzeigeelement die noch für die Ausschüttung dieser Dosis erforderliche Restmenge an.

In einer bevorzugten alternativen Variante kann die Antriebs- und Dosiervorrichtung so ausgestaltet sein, dass die für das Zurückdrehen des Dosisanzeigeelements oder/und das Verschieben des Vortriebsglieds in distale Richtung benötigte Energie automatisch, insbesondere durch eine Feder, insbesondere Ausschüttfeder, in der die benötigte Energie gespeichert ist oder werden kann, bereitgestellt wird. Z. B. kann die in der Ausschüttfeder gespeicherte Federenergie bei Betätigung des Betätigungsglieds an das Dosisanzeigeelement oder/und das Vortriebsglied abgegeben werden, so dass das Dosisanzeigeelement zurückgedreht und das Vortriebsglied in distale Richtung verschoben werden.

Die Feder kann bei Auslieferung der Antriebs- und Dosiervorrichtung bereits mit so viel Energie vorgespannt sein, dass die Energie für mehrere Ausschüttungen der Produktdosis ausreicht, insbesondere für die Ausschüttung des gesamten aus dem Produktbehälter ausschüttbaren Produkts ausreicht. In dieser Ausführung kann das Dosierglied bei der Dosiseinstellung von der Feder entkoppelt sein, d. h. nicht so mit der Ausschüttfeder gekoppelt sein, dass eine Drehung des Dosierglieds ein Spannen der Feder bewirkt. Hierdurch lässt sich das Dosierglied für den Verwender bei der Dosiseinstellung mit deutlich weniger Kraftaufwand drehen.

Das Dosierglied, insbesondere der Dosierknopf kann das Betätigungsglied, insbesondere den Betätigungsknopf umgeben oder aufnehmen. Somit können das Dosierglied und das Betätigungsglied das proximale Ende der Antriebs- und Dosiervorrichtung bilden. Vorzugsweise ist das Betätigungsglied für die Betätigung relativ zu dem Dosierglied verschiebbar.

Das Dosierglied kann relativ zu, insbesondere an dem Gehäuse axialfest und drehbar angeordnet sein.

Die Erfindung wurde anhand mehrerer vorteilhafter Ausführungsformen beschrieben. Im Folgenden werden vorteilhafte Ausführungsformen anhand von Figuren beschrieben. Die dabei offenbarten Merkmale bilden den Gegenstand der Ansprüche, insbesondere der unabhängigen Ansprüche, insbesondere einzeln und in jeglicher Kombination mit den Ansprüchen oder/und der vorhergehenden Beschreibung vorteilhaft weiter. Es zeigen:
- Figur 1: eine Explosionsdarstellung der Einzelteile einer beispielhaften Antriebs- und Dosiervorrichtung, bei der die Erfindung vorteilhaft eingesetzt werden kann,
- Figuren 2a-c: die Antriebs- und Dosiervorrichtung aus Figur 1 in einem Ausgangs- oder Auslieferungszustand, wobei Figur 2b eine Schnittansicht der Figur 2a ist und Figur 2c eine um 90° gedrehte Schnittansicht der Figur 2a entlang der Linie B-B ist,
- Figuren 3a-c: die Injektionsvorrichtung in den Ansichten aus Figur 2a in einem Zustand, in dem die maximal einstellbare Dosis eingestellt ist,
- Figuren 4a-c: die Ansichten aus Figuren 2a-c, jedoch in einem Zustand, bei dem die in den Figuren 3a-c eingestellte Dosis vollständig ausgeschüttet wurde und ein Betätigungsglied noch betätigt ist,
- Figuren 5a-c: die Ansichten aus den Figuren 2a-c, wobei ein Antriebsglied der Antriebs- und Dosiervorrichtung für eine Bewegung zur Dosiserhöhung gesperrt ist, weil die in dem Produktbehälter enthaltene Dosis geringer ist als die maximal einstellbare Dosis
- Figuren 6-9: verschiedene Ansichten einer ersten Ausführungsform der Erfindung,
- Figuren 10-14: verschiedene Ansichten einer zweiten Ausführungsform der Erfindung,
- Figuren 15-23: verschiedene Ansichten einer dritten Ausführungsform der Erfindung und
- Figuren 24-26: verschiedene Ansichten eines Gehäuseabschnitts und eines mehrteiligen Lagerelements einer vierten Ausführungsform der Erfindung.

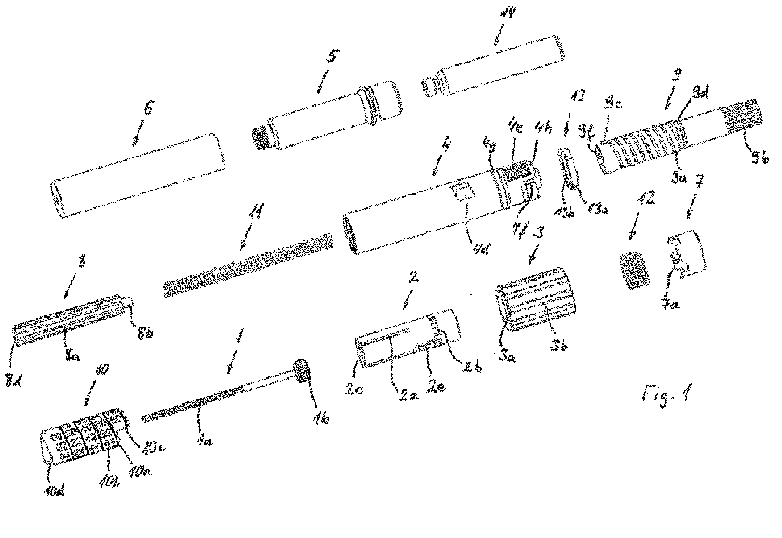

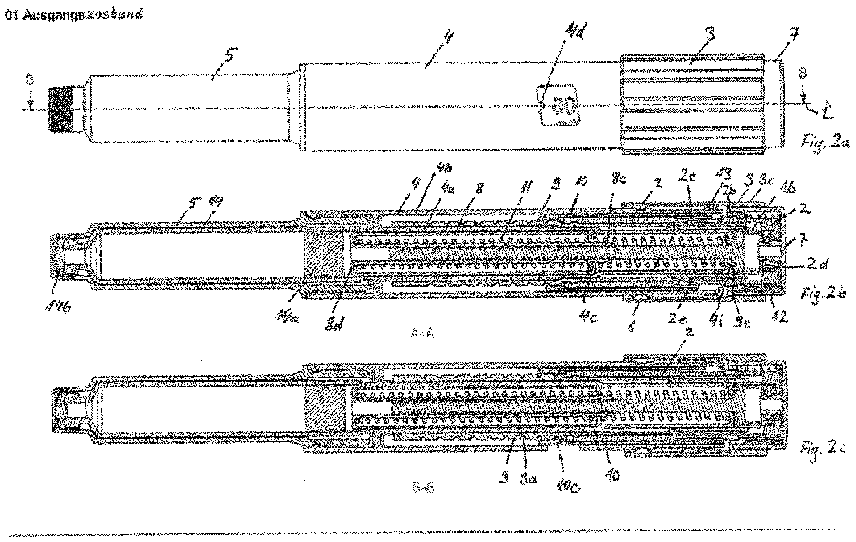

Eine Antriebs- und Dosiervorrichtung, bei welcher die Erfindung vorteilhaft einsetzbar oder integrierbar ist, kann, wie zum Beispiel aus den Figuren 1 und 2a bis 2c erkennbar ist, ein hülsenförmiges Gehäuse 4, welches eine Außenhülse 4b aufweist, welche vom Verwender mit einer Hand umgreifbar ist. Wie am besten aus Figur 2b erkennbar ist, umfasst das Gehäuse 4 ferner eine Innenhülse 4a, die ein Widerlager 4i bildet und konzentrisch zu der Außenhülse 4b angeordnet ist. Innenhülse 4a und Außenhülse 4b sind über einen Ringsteg miteinander verbunden. Zwischen der Außenhülse 4b und der Innenhülse 4a ist ein Ringspalt gebildet, in dem ein Dosisanzeigeelement 10, das insbesondere als Dosisanzeigetrommel, d. h. hülsenförmig ausgestaltet ist, ein Lagerelement 9 und ein Kupplungsglied 2, das hülsenförmig ist und insbesondere auch als Anzeigekupplung bezeichnet werden kann, angeordnet sind.

An dem distalen Ende des Gehäuses 4 ist eine hülsenförmige Produktbehälteraufnahme 5 aus einem vorzugsweise transparenten Material angeordnet, in der ein Produktbehälter 14 in der Gestalt einer Karpule aufgenommen ist. Der Produktbehälter 14 ist mittels der Produktbehälteraufnahme 5 mit dem Gehäuse 4 unlösbar verbunden, so dass die Antriebs- und Dosiervorrichtung insbesondere zusammen mit der Produktbehälteraufnahme 5 und dem Produktbehälter 14 eine Einweg-Injektionsvorrichtung bildet, die nach vollständiger Entleerung des Produktbehälters 14 als Ganzes entsorgt wird. Der Produktbehälter 14 weist an seinem distalen Ende ein Septum 14b auf, welches von einer durch am distalen Ende des Produktbehälters 14 bzw. der Produktbehälteraufnahme 5 anbringbaren Nadel durchstechbar ist. In dem Produktbehälter 14 ist ein Kolben 14a aufgenommen, wobei das auszuschüttende Produkt zwischen dem Septum 14b und dem Kolben 14a angeordnet ist. Eine Verschiebung des Kolbens 14a in Richtung Septum oder in distale Richtung, mithin Ausschüttrichtung, bewirkt eine Ausschüttung des in dem Produktbehälter 14 enthaltenen Produkts. In Figur 1 wird zusätzlich noch eine Schutzkappe 6 dargestellt, welche über der Produktbehälteraufnahme 5 anbringbar ist und vor dem Injizieren einer Dosis abgenommen wird.

Das Gehäuse 4, insbesondere die Innenhülse 4a ist in einem Eingriff mit einem hülsenförmigen Vortriebsglied 8, welches auch als Stößel bezeichnet werden kann. Das Vortriebsglied 8 ist relativ zu dem Gehäuse 4 drehfest und axial entlang der Längsachse L (Figur 2a) verschiebbar. Zwischen der Innenhülse 4a und dem Vortriebsglied 8 ist eine Führung mittels mindestens einer Längsrippe 8a und mindestens einer Längsführung 4c gebildet, welche eine Drehung des Vortriebsglieds 8 relativ zu dem Gehäuse 4 verhindert und eine Axialbewegung des Vortriebsglieds 8 relativ zu dem Gehäuse 4 erlaubt. Die Längsrippe 8a wird vorzugsweise von einer Außenhülse des Vortriebsglieds 8 gebildet. Das Vortriebsglied 8 weist eine Innenhülse 8b auf, welche in diesem Beispiel an ihrem proximalen Ende ein Innengewinde 8c aufweist, welches in ein Außengewinde 1a eines als Gewindestange ausgestalteten Rotationsglieds 1 eingreift. Das Vortriebsglied 8 ist so angeordnet, dass sein distales Ende 8d auf den Kolben 14a wirken, insbesondere gegen den Kolben 14a drücken kann.

Das Gehäuse 4, insbesondere das proximale Ende der Innenhülse 4a bildet das Widerlager 4i für eine Ausschüttfeder 11, die sich an dem Widerlager 4i und im Bereich des distalen Endes des Vortriebsglieds 8 abstützt. Die Feder 11 stützt sich mit ihrem distalen Ende an einem Ringsteg des Vortriebsglieds 8 ab, der die Außenhülse und die Innenhülse des Vortriebsglieds 8 verbindet. Die Feder 11 stützt sich mit ihrem proximalen Ende an dem von dem Gehäuse 4 gebildeten und nach innen ragenden Ringsteg ab, welcher das Widerlager 4i bildet.

Die Ausschüttfeder 11 ist als Schrauben- oder Wendelfeder gebildet, die als Druckfeder wirkt und danach strebt, das Widerlager 4i und das Vortriebsglied 8 auseinander zu drücken, d. h. das Vortriebsglied 8 in distale Richtung relativ zu dem Gehäuse 4 zu verschieben. Die Ausschüttfeder 11 ist bei der Auslieferung, d. h. im Ausgangszustand der Antriebs- und Dosiervorrichtung so stark vorgespannt, dass die in ihr gespeicherte Energie ausreicht, das in dem Produktbehälter 14 enthaltene Produkt im Wesentlichen vollständig auszuschütten, insbesondere mit mehreren Einzelausschüttungen, zwischen denen jeweils eine neue Dosiseinstellung vorgenommen wird. Der Vorteil an einer so stark vorgespannten Feder ist, dass die Feder 11 nicht z. B. während der Dosiseinstellung gespannt werden muss, wodurch für den Verwender der Vorrichtung eine Kraft sparende, d. h. einfachere Dosiseinstellung vornehmbar ist.

Der Gewindeeingriff zwischen dem Vortriebsglied 8 und dem Rotationsglied 1 ist so groß, dass keine Selbsthemmung des Gewindeeingriffs auftritt, d. h., dass das Rotationsglied 1 aufgrund der Axialkraft der Ausschüttfeder 11 relativ zu dem Vortriebsglied 8 um die Längsachse L drehbar oder rotierbar ist.

Das Rotationsglied 1 ist als Gewindestange ausgebildet, die das Außengewinde 1a bildet und an ihrem proximalen Ende einen vergrößerten Durchmesser, insbesondere in der Gestalt eines verbreiterten Kopfs aufweist. An dem Kopf sind parallel zur Längsachse L Zähne 1b angeordnet, die als zweite Kupplungsstruktur dienen, wie weiter unten beschrieben wird. An dem Kopf ist eine ringförmige, vom Durchmesser her gegenüber dem Kopf verkleinerte Reibfläche angeordnet, die in einem Kontakt mit dem nach innen ragenden, das Widerlager 4i bildenden Ringsteg des Gehäuses 4 ist. Durch den verringerten Durchmesser der ringförmigen Reibfläche wird der Angriffspunkt der resultierenden Reibkraft näher zur Längsachse L hin verschoben, wodurch das Reibmoment zwischen Rotationsglied 1 und Gehäuse 4 herabgesetzt wird.

Durch Drehung des Rotationsglieds 1 relativ zu dem Gehäuse 4 und dem Vortriebsglied 8 kann die Feder 11 das Vortriebsglied 8 um einen Ausschütthub in distale Richtung verschieben, der proportional zu dem Drehwinkel des Rotationsglieds 1 ist. Durch wahlweises Sperren und Freigeben des Rotationsglieds 1, was durch Betätigen eines als Betätigungsknopf ausgestalteten Betätigungsglieds 7 bewirkt werden kann, kann die Bewegung des Vortriebsglieds 8 relativ zu dem Gehäuse 4, d. h. der Ausschütthub des Vortriebsglieds 8 auf eine vorteilhafte Weise gesteuert werden.

Die Antriebs- und Dosiervorrichtung weist ferner ein Lagerelement 9 auf, welches auch als Anzeigetrommellagerelement bezeichnet werden kann und relativ zu dem Gehäuse 4 drehfest aber entlang der Längsachse L verschiebbar angeordnet ist. Das Lagerelement 9 erfüllt im gezeigten Beispiel die Aufgabe des hierin beschriebenen Verschiebeglieds. Das Lagerelement 9 ist hülsenförmig und umgibt vorzugsweise die Innenhülse 4a des Gehäuses 4, wobei insbesondere die Außenhülse 4b das Lagerelement 9 umgibt. Das Lagerelement 9 ist in einem Eingriff mit dem Gehäuse 4, insbesondere der Innenhülse 4a, der eine Längsbewegung des Lagerelements 9 relativ zu dem Gehäuse 4 zulässt, aber eine Drehbewegung verhindert. Der Eingriff kann durch eine Längsführung 9f zwischen dem Lagerelement 9 und der Innenhülse 4a gebildet werden.

Das Lagerelement 9 weist ein Gewinde 9a, insbesondere ein Außengewinde auf, in welches ein Gewinde 10e, insbesondere ein Innengewinde, des Dosisanzeigeelements 10 eingreift. Durch diesen Gewindeeingriff ist das Anzeigeelement 10 relativ zu dem Lagerelement 9 schraubbar.

Die erste Ausführungsform umfasst ferner einen Signalerzeugungsmechanismus 2e, 9b, der ein akustisches und/oder taktiles Signal bei der Dosiseinstellung und der Produktausschüttung erzeugt. Der Signalerzeugungsmechanismus 2e, 9b ist zwischen dem Kupplungsglied 2 und dem Lagerelement 9 angeordnet und umfasst insbesondere ein Rastglied 2e und eine Verzahnung 9b. Das Lagerelement 9 weist eine sich über den Umfang, insbesondere Außenumfang erstreckende Verzahnung 9b auf. Das Kupplungsglied 2 weist das federnd angeordnete und in die Verzahnung 9b eingreifende Rastglied 2e auf.

Das Lagerelement 9 weist an seinem proximalen Ende die über seinen Umfang erstreckte Verzahnung 9b auf, die z. B. zur Einstellung von diskreten dosisproportionalen Winkelschritten und/oder zur Erzeugung eines leichten Widerstands bei der Dosiseinstellung und/oder zur Erzeugung des akustischen oder taktilen Signals, wie z. B. eines hör- und fühlbaren Klicks bei der Dosiseinstellung und der Produktausschüttung, dient. In die Verzahnung 9b greifen zwei Rastglieder 2e ein, welche federnd an Rastarmen angeordnet sind und von dem Kupplungsglied 2 gebildet werden. Das Kupplungsglied 2 ist axialfest mit dem Lagerelement 9 und relativ zu dem Lagerelement 9 drehbar verbunden. Hierzu greift das Kupplungsglied 2 mittels einer Ringnut 2c in eine über den Umfang des Lagerelements 9 sich erstreckende ringförmige Abragung 9d ein. Eine Drehung des hülsenförmigen Kupplungsglieds 2 relativ zu dem Lagerelement 9 bewirkt, dass die Rastglieder 2e über die Verzahnung 9b rasten und dabei das akustische und/oder taktile Signal erzeugen.

Das Dosisanzeigeelement 10 ist drehfest, aber axial verschiebbar mit dem Kupplungsglied 2 verbunden, insbesondere in einem Eingriff. Dieser Eingriff umfasst eine Längsführung 2a, welche bewirkt, dass das Dosisanzeigeelement 10 relativ zu dem Kupplungsglied 2 drehfest, aber axial verschiebbar ist. Eine Drehung des Kupplungsglieds 2 relativ zu dem Lagerelement 9 bewirkt aufgrund der drehfesten Verbindung zwischen Kupplungsglied 2 und Dosisanzeigeelement 10, dass das Dosisanzeigeelement 10 ebenfalls gedreht und aufgrund des Gewindeeingriffs in das Gewinde 9a an dem Lagerelement 9 entlang geschraubt wird, insbesondere zusätzlich zu dem aufgrund der Rastglieder 2e erzeugten Klickgeräusche.

Das Dosisanzeigeelement 10 weist über seinen Außenumfang eine sich entsprechend der Steigung des Gewindes 10e wendelförmig ersteckende Dosisskala 10b auf, die eine Vielzahl nacheinander angeordnete Skalenwerte umfasst. In dem gezeigten Beispiel kann mit der Antriebs- und Dosiervorrichtung eine maximale Dosis von 80 I.U. eingestellt werden, wobei die Skala von 0 bis 80 reicht und die Dosiswerte in Zweierschritten angegeben sind.

Ebenfalls entsprechend der Steigung des Gewindes 10e ist eine Markierung 10a wendelförmig über den Außenumfang des Dosisanzeigeelements 10 angeordnet. Diese Markierung 10a dient - wie weiter unten beschrieben wird - zur Anzeige, ob die Vorrichtung betätigt oder unbetätigt ist. Die Markierung 10a ist eine optionale Einrichtung. Sie kann sich entlang der ganzen Dosisskala 10b oder nur Teilen oder einem einzelnen Skalenwert davon erstrecken. Insbesondere kann sie bei betätigter Antriebs- und Dosiervorrichtung nur gegen Ende der Produktausschüttung oder in der Nulldosisposition sichtbar sein.

Das Dosisanzeigeelement 10 weist an seinem z. B. proximalen Ende eine in Umfangsrichtung weisende und wirkende Anschlagfläche 10c auf, die als Nulldosisanschlag bezeichnet wird. Das Dosisanzeigeelement 10 weist an seinem, z. B. dem proximalen Ende entgegengesetzten, distalen Ende eine in Umfangsrichtung weisende und wirkende Anschlagfläche 10d auf, welche als Maximaldosisanschlag bezeichnet wird.

Das Dosisanzeigeelement 10 ist an dem Lagerelement 9 zwischen einer Nulldosisposition und einer Maximaldosisposition hin und her schraubbar. In der Nulldosisposition verhindert der Nulldosisanschlag 10c im Zusammenwirken mit einem von dem Gehäuse 4 gebildeten Nulldosisgegenanschlag 4f die Drehung des Dosisanzeigeelements 10 in eine erste Drehrichtung, nämlich eine Drehrichtung, die bewirken würde, dass eine kleinere Dosis als Null eingestellt wird. In dieser Nulldosisposition ist das Dosisanzeigeelement 10 in die entgegengesetzte, d. h. zweite Drehrichtung drehbar.

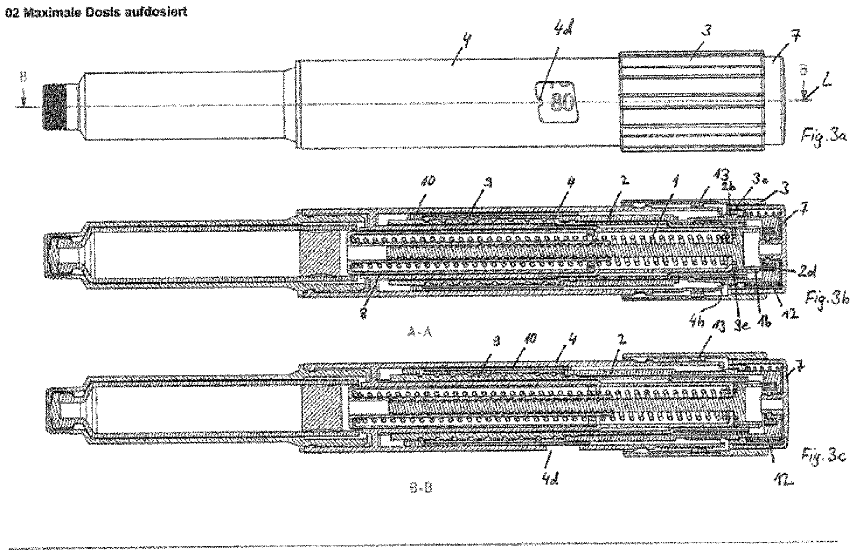

In der Maximaldosisposition, die z. B. in Figur 3a gezeigt wird, verhindert der Maximaldosisanschlag 10d im Zusammenwirken mit einem Maximaldosisgegenanschlag 9c, der von dem Lagerelement 9 gebildet wird, die Drehung des Dosisanzeigeelements 10 in die zweite Drehrichtung, welche eine Erhöhung der Dosis über den maximal einstellbaren Wert hinaus bewirken würde. Die Drehung in die erste Drehrichtung ist in der Maximaldosisposition möglich. Obgleich der Maximaldosisgegenanschlag 9c von dem Lagerelement 9 gebildet wird, kann abweichend von diesem Beispiel der Maximaldosisgegenanschlag 9c optional von dem Gehäuse 4 gebildet werden. Der Nulldosisgegenanschlag kann abweichend von dem gezeigten Beispiel von dem Lagerelement 9 gebildet werden.

Das Gehäuse 4 weist eine Zeigeeinrichtung 4d in der Gestalt eines Fensters auf, welches den Blick auf die Skala 10b des Dosisanzeigeelements 10 frei gibt. An dem Gehäuse 4 ist ein Dosierglied 3 in der Gestalt eines Dosierknopfs drehbar aber axialfest gelagert. Hierfür weist das Gehäuse 4 eine Ringnut 4g auf, in welche insbesondere eine Ringschulter des Dosierglieds 3 eingreift. Das Dosierglied 3 weist über seinen Außenumfang eine Griffstruktur 3b auf, welche es dem Verwender der Vorrichtung erleichtert, das Dosierglied 3 relativ zu dem Gehäuse 4 zu verdrehen. Im unbetätigten Zustand der Vorrichtung bewirkt eine Drehung des Dosierglieds 3 eine Drehung oder Schraubbewegung des Dosisanzeigeelements 10, wodurch die gewünschte Dosis einstellbar und in der Zeigeeinrichtung 4d ablesbar ist.

An dem Dosierglied 3 ist ein Betätigungsglied 7 in der Gestalt eines Betätigungsknopfs angeordnet, welches relativ zu dem Dosierglied 3 für eine Betätigung der Vorrichtung zur Produktausschüttung bewegbar ist, insbesondere entlang der Längsachse L. Das Betätigungsglied 7 bildet das proximale Ende der Vorrichtung und ist vom Daumen der Hand des Verwenders, welche das Gehäuse 4 umgreift, auf einfache Weise betätigbar, insbesondere relativ zu dem Gehäuse 4 und/oder zu dem Dosierglied 3 verschiebbar. Das Kupplungsglied 2 ist relativ zu dem Betätigungsglied 7, insbesondere bei gelöster Dosierkupplung 2b, 3c, drehbar und axialfest. Vorzugsweise ist das Betätigungsglied 7 mit dem Kupplungsglied 2 axialfest aber drehbar verschnappt.

Die Antriebs- und Dosiervorrichtung weist ferner eine Rücksetz- oder Kupplungsfeder 12 auf, die beim Betätigen, insbesondere Drücken des Betätigungsglieds 7 gespannt wird und die das Lagerelement 9 und/oder das Betätigungsglied 7 in seine unbetätigte Position zurücksetzt, wenn das Betätigungsglied 7 unbetätigt ist. Eine Betätigung des Betätigungsglieds 7 bewirkt neben seiner axialen Verschiebung ebenfalls die axiale Verschiebung des Lagerelements 9 entlang der Längsachse L. Die Feder 12 stützt sich mit ihrem distalen Ende vorzugsweise an dem Dosierglied 3 und mit ihrem proximalen Ende vorzugsweise an dem Betätigungsglied 7 ab. Die Feder 12 ist vorzugsweise eine Schrauben- oder Wendelfeder, welche als Druckfeder wirkt.

Das Dosierglied 3 ist relativ zu dem Betätigungsglied 7 drehfest. Das Betätigungsglied 7 durchgreift eine nach innen ragende Schulter des Dosierglieds 3. An dem distalen Ende des vorzugsweise topfförmig ausgestalteten Betätigungsglieds 7 sind mehrere Zähne gebildet, die zusammen eine Verzahnung 7a bilden, welche durch Betätigung des Betätigungsglieds 7 mit einer an dem Gehäuse 4 gebildeten Verzahnung 4h, insbesondere am proximalen Ende des Gehäuses 4, in einen Eingriff geraten, wodurch das Dosierglied 3 in Bezug auf das Gehäuse 4 drehfest ist. Dies bewirkt, dass bei betätigter Vorrichtung eine Dosiseinstellung, d. h. eine Verdrehung des Dosierglieds 3 relativ zu dem Gehäuse 4 nicht möglich ist, sondern erst dann, wenn das Betätigungsglied 7 unbetätigt ist.

Das Dosierglied 3 bildet eine Kupplungsstruktur 3c, insbesondere an der nach innen ragenden Schulter. Die Kupplungsstruktur 3c wirkt bei unbetätigtem Betätigungsglied 7 mit einer Kupplungsstruktur 2b am äußeren Umfang des Kupplungsglieds 2 zusammen. Im unbetätigten Zustand des Betätigungsglieds 7 sind das Dosierglied 3 und das Kupplungsglied 2 relativ zueinander drehfest aufgrund dieses Kupplungseingriffs. Die Kupplung zwischen Dosierglied 3 und Kupplungsglied 2 kann auch als Dosierkupplung 2b, 3c bezeichnet werden, die bei der Dosiseinstellung, d. h. bei unbetätigtem Betätigungsglied 7 eingerückt und bei der Dosisausschüttung, d. h. bei betätigtem Betätigungsglied 7 ausgerückt ist, wobei die Kupplung im eingerückten Zustand Drehmoment überträgt und im ausgerückten Zustand kein Drehmoment überträgt. Die Dosierkupplung 2b, 3c wird durch eine Verschiebung des Kupplungsglieds 2 relativ zu dem Gehäuse 4 ausgerückt oder geöffnet, insbesondere aufgrund der Betätigung des Betätigungsglieds 7.

Das Lagerelement 9 weist an seinem proximalen Ende am Innenumfang eine erste Kupplungsstruktur 9e auf, welche durch über den Umfang angeordnete Klauen oder Zähne gebildet werden, die mit den die zweite Kupplungsstruktur 1b bildenden Zähnen oder Klauen des Rotationsglieds 1 in einem Eingriff sind, insbesondere bei unbetätigtem Betätigungsglied 7. Durch diesen Kupplungseingriff ist das Rotationsglied 1 in Bezug auf das Gehäuse 4 drehfest. Das Kupplungsglied 2 weist ferner eine dritte Kupplungsstruktur 2d an einem Innenumfang auf, die mehrere über den Umfang verteilte Zähne oder Klauen aufweist. Die dritte Kupplungsstruktur 2d ist so angeordnet, dass sie bei Betätigung des Betätigungsglieds 7 in einen drehfesten Eingriff mit dem Rotationsglied 1 gelangt, insbesondere mit der zweiten Kupplungsstruktur 1b oder alternativ einer von der zweiten Kupplungsstruktur 1b separaten, in diesem Beispiel jedoch nicht gezeigten, vierten Kupplungsstruktur.

Während das Betätigungsglied 7 für die Betätigung relativ zu dem Dosierglied 3 entlang der Längsachse L verschoben wird, gerät zunächst die dritte Kupplungsstruktur 2d in einen Eingriff mit der zweiten Kupplungsstruktur 1b. Durch weiteres Verschieben des Betätigungsglieds 7 relativ zu dem Dosierglied 3 gerät die erste Kupplungsstruktur 9e aus dem Eingriff mit der zweiten Kupplungsstruktur 1b. Bevor, nachdem oder gleichzeitig mit dem Lösen des Eingriffs der ersten Kupplungsstruktur 9e mit der zweiten Kupplungsstruktur 1b gerät die Kupplungsstruktur 2b mit der Kupplungsstruktur 3c außer Eingriff und/oder die Verzahnung 7a mit der Verzahnung 4h in einen Eingriff.

Insbesondere dadurch, dass die erste Kupplungsstruktur 9e von der zweiten Kupplungsstruktur 1b gelöst ist, kann die Ausschüttfeder 11 sich entspannen, wobei das Rotationsglied 1 relativ zu dem Gehäuse 4 verdreht wird und aufgrund des Eingriffs der zweiten Kupplungsstruktur 1b mit der dritten Kupplungsstruktur 2d das Kupplungsglied 2 und somit auch das Dosisanzeigeelement 10 relativ zu dem Gehäuse 4 verdreht werden, wodurch das Dosisanzeigeelement 10 in seine Nulldosisposition zurück geschraubt und das Vortriebsglied 8 proportional zu dem sich insbesondere in Umfangsrichtung erstreckenden Abstand zwischen Nulldosisanschlag 10c und Nulldosisgegenanschlag 4f um einen Ausschütthub relativ zu dem Gehäuse 4 in distale Richtung verschoben wird. Die Drehung des Kupplungsglieds 2 relativ zu dem Lagerelement 9 bewirkt, dass die Rastglieder 2e über die Verzahnung 9b insbesondere in dosisproportionalen Winkelschritten rasten und dabei das akustische und /oder taktile Signal erzeugen.

Die Antriebs- und Dosiervorrichtung weist einen Dosisbegrenzer 13 in der Gestalt eines Rings, eines Ringsegments oder einer Mutter auf, der an seinem Innenumfang ein Gewinde 13b aufweist, das in ein an einem Außenumfang des Gehäuses 4 angeordnetes Gewinde 4e eingreift, so dass der Begrenzer 13 relativ zu dem Gehäuse 4 schraubbar ist. Am Außenumfang weist der Begrenzer 13 ein Eingriffsglied 13a auf, welches in eine Längsführung 3a am Innenumfang des Dosierglieds 3 eingreift, so dass das der Dosisbegrenzer 13 relativ zu dem Dosierglied 3 drehfest aber axial verschiebbar ist. An dem Dosierglied 3 oder dem Gehäuse 4 ist ein Stoppanschlag gebildet, von dem der Begrenzer 13 proportional zu der maximal aus dem Produktbehälter 14 ausschüttbaren Produktmenge beabstandet ist. Da bei der Dosiseinstellung das Dosierglied 3 relativ zu dem Gehäuse 4 verdreht und bei einer Dosisausschüttung nicht verdreht wird, kann durch den Begrenzer 13 ein Zählwerk gebildet werden, welches die bereits ausgeschütteten Einzeldosen und die aktuell eingestellte Dosis addiert und sich dementsprechend immer näher an den Stoppanschlag des Dosierglieds 3 oder des Gehäuses 4 annähert. Eine Dosiserhöhung bewirkt, dass der Begrenzer 13 zu dem Stoppanschlag hin bewegt wird. Eine Dosisverringerung bewirkt, dass der Begrenzer 13 von dem Stoppanschlag weg bewegt wird. Ist die in dem Produktbehälter 14 angegebene Restdosis geringer als die maximal mit der Antriebs- und Dosiervorrichtung einstellbare Dosis, gerät der Begrenzer 13 in einen Kontakt mit dem Stoppanschlag, so dass eine Verdrehung des Dosierglieds 3 relativ zu dem Gehäuse 4 in eine Drehrichtung, die eine Erhöhung der Dosis zur Folge hätte, blockiert wird.

Die aus der ersten, zweiten und dritten sowie optional der vierten Kupplungsstruktur 9e, 1b, 2d gebildete Kupplung kann aufgrund ihres Zusammenspiels auch als Ausschüttkupplung bezeichnet werden.

In den Figuren 2a bis 2c wird die Antriebs- und Dosiervorrichtung, die auch als Injektionsvorrichtung bezeichnet werden kann, in ihrem Ausgangs- oder Auslieferungszustand, insbesondere vor einer ersten Verwendung gezeigt. Die in der Zeigeeinrichtung 4d angezeigte Produktdosis ist 0. Ein Betätigen des Betätigungsglieds 7 hätte zur Folge, dass keine Dosis ausgeschüttet wird. Der Begrenzer 13 weist einen Abstand zu dem Stoppanschlag auf, welcher proportional zu der in dem Produktbehälter 14 enthaltenen oder ausschüttbaren Produktmenge ist, wie z. B. 300 I.U.

Zur Einstellung der Produktdosis wird das Dosiseinstellglied 3 relativ zu dem Gehäuse 4 verdreht, wodurch aufgrund des Kupplungseingriffs 2b, 3c das Kupplungsglied 2 und somit auch das Dosisanzeigeelement 10 relativ zu dem Gehäuse 4 verdreht werden. Dabei schraubt sich das Dosisanzeigeelement 10 entlang dem Lagerelement 9 aufgrund des Gewindeeingriffs des Gewindes 10e mit dem Gewinde 9a. Insbesondere wird der Abstand zwischen dem Nulldosisanschlag 10c und dem Nulldosisgegenanschlag 4f proportional zu der in der Zeigeeinrichtung 4d gezeigten Dosis erhöht. Außerdem wird bei der Drehung aufgrund des Überrastens der Rastglieder 2e über die Verzahnung 9b ein hörbares und fühlbares Signal erzeugt.

In den Figuren 3a bis 3c wird die Antriebs- und Dosiervorrichtung in einem Zustand gezeigt, in der eine maximal einstellbare Dosis eingestellt wurde, nämlich in diesem Beispiel 80 I.U., die in der Zeigeeinrichtung 4d ablesbar sind. Eine weitere Dosiserhöhung ist aufgrund des Zusammenwirkens, insbesondere des Kontakts des Maximaldosisanschlags 10d mit dem Maximaldosisgegenanschlag 9c nicht möglich. Der Dosisbegrenzer 13 ist, wie am besten aus den Figuren 3b und 3c erkennbar ist, entsprechend 80 I.U. weit an den Stoppanschlag herangerückt oder verschoben.

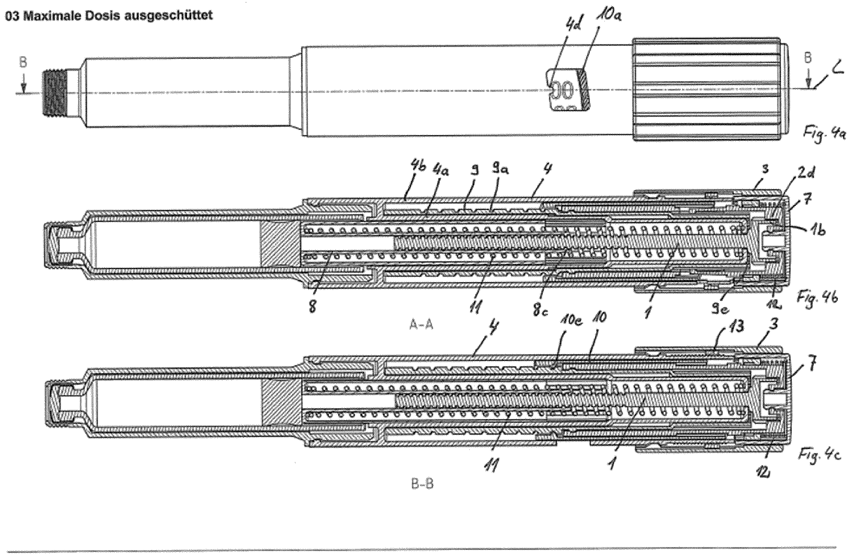

Zur Ausschüttung der z. B. in Figur 3a angezeigten Dosis wird das Betätigungsglied 7 betätigt, insbesondere gedrückt, d. h. relativ zu dem Gehäuse 4 und dem Dosierglied 3 in distale Richtung verschoben, wodurch das Kupplungsglied 2 und das Lagerelement 9 sowie das Dosisanzeigeelement 10 relativ zu dem Gehäuse 4 in distale Richtung verschoben werden, insbesondere gegen die Kraft der Kupplungs- oder Rücksetzfeder 12. Dadurch, dass das Dosisanzeigeelement 10 relativ zu dem Gehäuse 4 und der Zeigeeinrichtung 4d axial verschoben wird, erscheint die in Figur 1 gezeigte Markierung 10a in der Zeigeeinrichtung 4d (Figur 4a), wodurch der Verwender optisch ablesen kann, dass die Vorrichtung betätigt ist. Durch die Verschiebung des Dosisanzeigeelements 10 relativ zu dem Gehäuse 4 und der Zeigeeinrichtung 4d wird die Markierung 10a aus einer Position, in der sie von dem Gehäuse 4 verdeckt wird, in eine Position, in der sie in der Zeigeeinrichtung 14d gezeigt wird, entlang der Längsachse L verschoben.

Die Betätigung des Betätigungsglieds 7 bewirkt ferner, dass die dritte Kupplungsstruktur 2d mit der zweiten Kupplungsstruktur 1b einrückt und die erste Kupplungsstruktur 9e von der zweiten Kupplungsstruktur 1b ausrückt, so dass das Rotationsglied 1 in Bezug auf das Gehäuse 4 nicht mehr drehfest sondern drehbar ist und in Bezug auf das Kupplungsglied 2 und das Dosisanzeigeelement 10 drehfest ist. Die Betätigung des Betätigungsglieds 7 bewirkt ferner, dass die Dosierkupplung 2b, 3c ausrückt bzw. geöffnet wird und die Stirnverzahnung 7a in den Eingriff mit der Stirnverzahnung 4h gerät. Im betätigten Zustand des Betätigungsglieds 7 ist das Rotationsglied 1 relativ zu dem Dosisanzeigeelement 10 drehfest, wodurch sich das Rotationsglieds 1 und das Dosisanzeigeelement 10 gemeinsam relativ zu dem Gehäuse 4 drehen können. Durch die Kraft der in der Ausschüttfeder 11 gespeicherten Energie auf das Vortriebsglied 8 wird aufgrund des Gewindeeingriffs des Vortriebsglieds 8 mit dem Rotationsglied 1 eine Drehung des Rotationsglieds 1 und des Dosisanzeigeelements 10 relativ zu dem Gehäuse 4 bewirkt, wodurch sich das Dosisanzeigeelement 10 an dem Lagerelement 9 in Richtung Nulldosisposition zurückschraubt und wobei die in der Zeigeeinrichtung 14d angezeigte Dosis runterzählt. Gleichzeitig wird das Vortriebsglied 8 von der Ausschüttfeder 11 relativ zu dem Gehäuse 4 in distale Richtung um den Ausschütthub bewegt, welcher proportional zu der zuvor eingestellten Dosis ist. Wenn das Dosisanzeigeelement 10 seine Nulldosisposition erreicht hat (Figuren 4a bis 4c) ist die zuvor eingestellte Dosis oder Einzeldosis ausgeschüttet. Lässt der Verwender das in den Figuren 4a bis 4c noch als gedrückt dargestellte Betätigungsglied 7 los, setzt die Kupplungs- oder Rücksetzfeder 12 das Betätigungsglied 7, das Kupplungsglied 2, das Lagerelement 9 und das Dosisanzeigeelement 10 in die z. B. in Figur 2a gezeigte Position zurück, wobei die Markierung 10a wieder unter dem Gehäuse 4 verschwindet oder von dem Gehäuse 4 verdeckt wird. Beim Rücksetzen werden die genannten Elemente relativ zu dem Gehäuse 4 oder dem Dosierglied 3 in proximale Richtung verschoben.

Beim Zurücksetzen der Vorrichtung mittels der Feder 12 wird die erste Kupplungsstruktur 9e mit der zweiten Kupplungsstruktur 1b eingerückt und die dritte Kupplungsstruktur 2d von der zweiten Kupplungsstruktur 1b ausgerückt. Das Rotationsglied 1 ist nun wieder drehfest in Bezug auf das Gehäuse 4, wobei das Dosierglied 3 zusammen mit dem Dosisanzeigeelement 10 relativ zu dem Gehäuse 4 und/oder der Zeigeeinrichtung 4d und/oder dem Rotationsglied 1 für eine neue Einstellung einer Produktdosis oder Einzeldosis drehbar ist. Beim Zurücksetzen werden ferner die Stirnverzahnungen 7a und 4h aus dem Eingriff gelöst sowie die Dosierkupplung 2b, 3c wieder eingerückt, wodurch das Dosierglied 3 relativ zu dem Kupplungsglied 2 und dem Dosisanzeigeelement 10 drehfest ist.

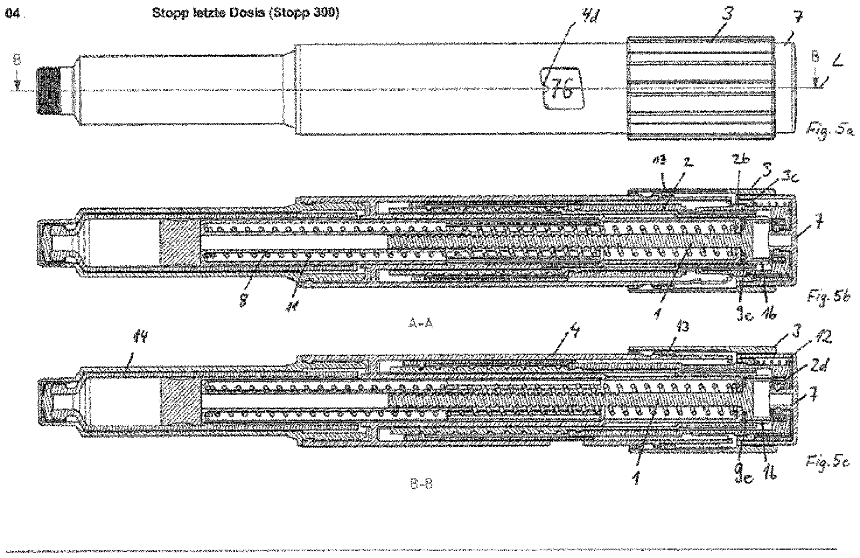

In Figur 5a wird die Antriebs- und Dosiervorrichtung in einer Position gezeigt, in welcher der Begrenzer 13 seine Stoppposition einnimmt, d. h. an dem Stoppanschlag anschlägt, wodurch der Begrenzer 13 eine Dosiseinstellung auf einen Wert, welcher die in dem Produktbehälter 14 enthaltene Restmenge übersteigt, blockiert. In dem gezeigten Beispiel sind noch 76 I.U. in dem Produktbehälter 14 enthalten, wobei mit der Antriebs- und Dosiervorrichtung maximal 80 I.U. einstellbar wären. Da der Begrenzer 13 bereits bei 76 I.U. in einem Kontakt mit dem Stoppanschlag ist, wird das Dosierglied 3 für eine Drehung in die zweite Richtung, welche eine Erhöhung der Dosis bewirken würde, gesperrt. Die Verringerung der Dosis ist jedoch durch Drehen des Dosierglieds 3 in die erste Drehrichtung möglich.

Durch Betätigen des Betätigungsglieds 7 wird die in der Zeigeeinrichtung 4d gezeigte Dosis ausgeschüttet. Da anschließend der Produktbehälter 14 vollständig entleert ist, wird die Antriebs- und Dosiervorrichtung oder Injektionsvorrichtung als Ganzes entsorgt. Es handelt sich somit um eine Einweg-Injektionsvorrichtung. Grundsätzlich können die hierin gezeigten Antriebs- und Dosiervorrichtungen aber auch in Verbindung mit Mehrweginjektionsvorrichtungen Anwendung finden, bei denen ein entleerter Produktbehälter 14 gegen einen neuen ausgetauscht wird.

Die mit Bezug auf die Figuren 1 bis 5c beschriebene Antriebs- und Dosiervorrichtung ist aus der europäischen Patentanmeldung Nr. 12 162 777.2 bekannt. Um sicherzustellen, dass das Rotationsglied 1 bei vollständig betätigtem Betätigungsglied auch tatsächlich seine Drehbewegung für die Ausschüttung ausführt, ist das Betätigungsglied 7 so mit dem Rotationsglied 1 gekoppelt, dass das Betätigen oder Drücken des Betätigungsglieds 7 bewirkt, dass sich das Rotationsglied 1 relativ zu dem Gehäuse 4 bereits dann dreht, wenn die Kupplung 9e, 1b noch geschlossen ist. Zumindest das Rotationsglied 1 wird hierdurch bereits in eine Drehbewegung versetzt, so dass Haftreibungen in der Antriebs- und Dosiervorrichtung überwunden werden, insbesondere auch dann, wenn die Teile aufgrund von längerer Lagerung "fest" geworden sind.

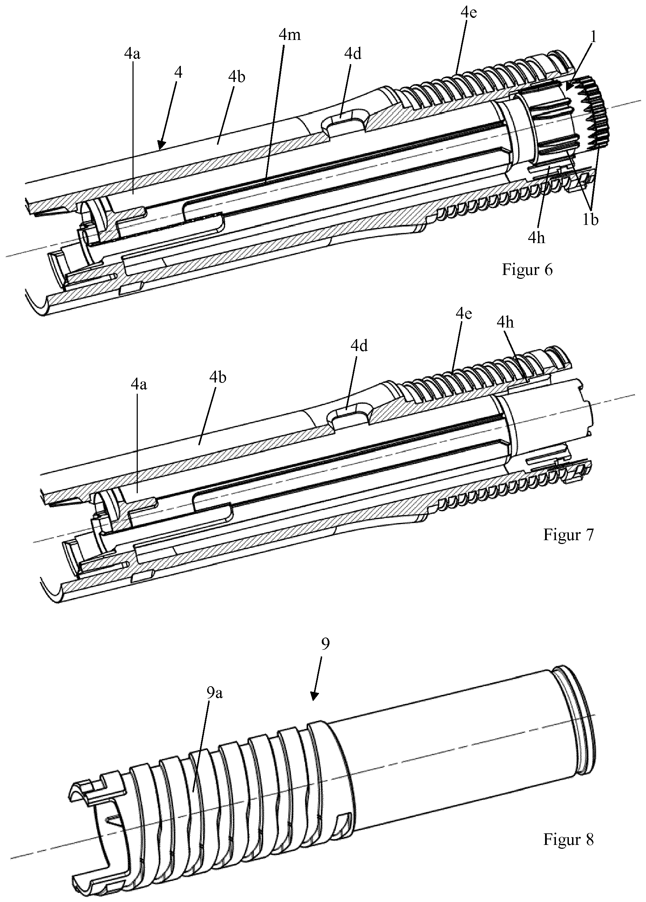

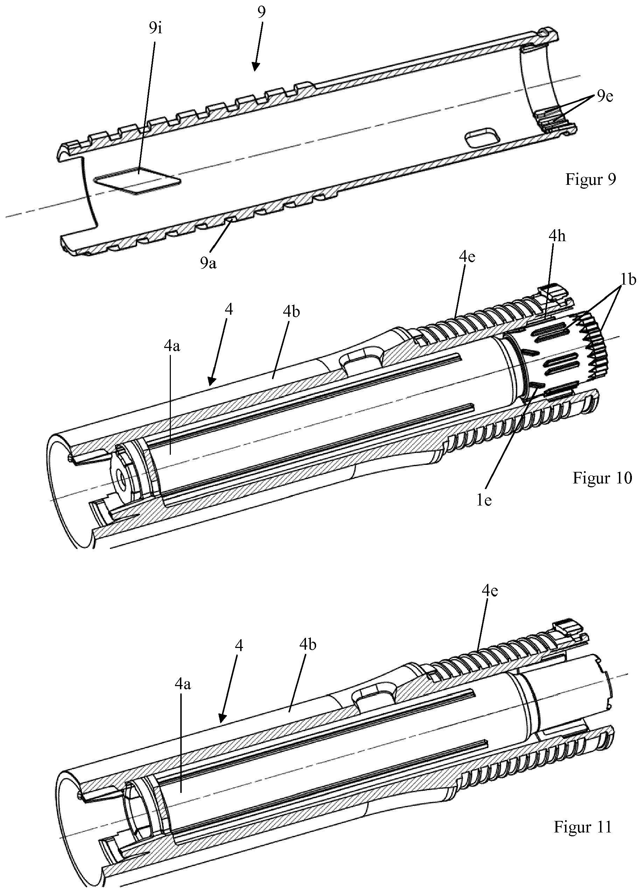

In der ersten Ausführungsform weist die Innenhülse 4a eine Führungsbahn 4m, die insbesondere nutförmig ausgestaltet ist, auf Die längliche, sich entlang, insbesondere parallel zur Längsachse L erstreckende Führungsbahn 4m weist einen proximalen Führungsbahnabschnitt und einen distalen Führungsbahnabschnitt auf, die mit einem Übergangsabschnitt miteinander verbunden sind, insbesondere ineinander münden, wie am besten aus den Figuren 6 und 7 ersichtlich ist. Der proximale Führungsbahnabschnitt 4m ist in Bezug auf den distalen Führungsbahnabschnitt um die Längsachse L winkelversetzt angeordnet. Die nutförmige Führungsbahn 4m ist zumindest nach außen oder zum Lagerelement 9 hin offen, wobei eine Abragung 9i (Figur 9) am Innenumfang des Lagerelements 9 in die Führungsbahn 4m eingreift. Beispielsweise kann die Form der Abragung 9i so an den Übergangsbereich zwischen dem proximalen und dem distalen Führungsbahnabschnitt angepasst sein, dass bei einer Verschiebung der Abragung 9i in die distale Richtung die Abragung 9i so von dem proximalen Führungsbahnabschnitt in den distalen Führungsbahnabschnitt, insbesondere durch den Übergangsabschnitt geführt wird, dass das Lagerelement 9 eine Drehbewegung relativ um die Längsachse L ausführt. Da in dem gezeigten Beispiel die Abragung 9i fest an dem Lagerelement 9 gebildet ist, wird die durch das Verschieben der Abragung 9i auf sie ausgeübte Drehbewegung auf das Lagerelement 9 übertragen.

Die Abragung 9i kann zum Beispiel rund, insbesondere kreisrund sein, wobei der Übergangsbereich zwischen proximalen und distalen Führungsbahnabschnitten abgeschrägt oder gewindeförmig sein kann (nicht gezeigt). In der gezeigten Ausführung ist die Abragung 9i eine rautenförmige Nocke 9i. Das in die distale Richtung weisende Ende der rautenförmigen Nocke 9i wird beim Betätigen des Bestätigungsglieds 7 durch Verschiebung des Lagerelements 9 in die distale Richtung aus dem proximalen Führungsbahnabschnitt zumindest teilweise in den distalen Führungsbahnabschnitt verschoben, wobei sich der proximale Führungsbahnabschnitt und der distale Führungsbahnabschnitt der Führungsbahn 4m im Übergangsbereich bezogen auf die Winkelposition um die Längsachse L einander überlappen. Die zwischen den zur Längsachse L parallelen Flächen angeordnete schräge Fläche der rautenförmigen Nocke, die in die distale Richtung weist, gleitet an der distalen Kante, die zwischen dem proximalen und dem distalen Führungsbahnabschnitt gebildet ist, ab, so dass die rautenförmige Nocke 9i und somit das Lagerelement 9 in eine Drehbewegung in die erste Drehrichtung um die Längsachse L versetzt werden.

Die rautenförmige Nocke 9i weist zwei parallele in Umfangsrichtung weisende Flächen auf, die parallel zur Längsachse L angeordnet sind. Diese Flächen weisen einen Abstand zueinander auf, der in etwa der Breite der Führungsbahn 4m, insbesondere des proximalen und/oder distalen Führungsbahnabschnitts entspricht. Die schrägen, in die distale Richtung und die proximale Richtung weisenden, zueinander parallelen Flächen, welche die parallel zur Längsachse L erstreckten Flächen der rautenförmigen Nocke 9i verbinden, können zum Beispiel als Getriebeflächen wirken.

Das Lagerelement 9 weist eine erste Kupplungsstruktur 9e auf, welche als Innenverzahnung ausgestaltet ist und in die zweite Kupplungsstruktur 1b, des Rotationsglieds 1, die als Außenverzahnung gebildet ist, verdrehfest eingreift, wenn das Betätigungsglied 7 unbetätigt ist bzw. nicht in seiner (vollständig) betätigten Position ist. Die erste Kupplungsstruktur 9e und die Abragung 9i sind so zueinander angeordnet, dass das Lagerelement 9 bereits dann in die erste Drehrichtung gedreht wird, wenn die Kupplung 9e, 1b noch geschlossen ist, wodurch das Rotationsglied 1 mitgedreht wird. Dies geschieht in einer Zwischenposition des Betätigungsglieds 7 zwischen der unbetätigten Position und betätigten Position. Wird das Betätigungsglied 7 aus der Zwischenposition in die betätigte Position gebracht, wird die Kupplung 9e, 1b geöffnet, wodurch das Rotationsglied 1 von der Ausschüttfeder 11 rotatorisch angetrieben wird.

Wenn das Betätigungsglied 7 losgelassen wird, setzt die Feder 12 das Betätigungsglied 7 in seine unbetätigte Position zurück, wobei die schräge, in die proximale Richtung weisende Fläche, der Nocke 9i an der proximalen Kante, die zwischen dem proximalen und dem distalen Führungsbahnabschnitt gebildet wird, ab, so dass die Nocke 9i und somit das Lagerelement 9 in eine Drehbewegung in die zweite Drehrichtung um die Längsachse L versetzt werden. Die Kupplung 9e, 1b ist hierbei geschlossen, so dass das Rotationsglied 1 ebenfalls in die zweite Drehrichtung gedreht wird, wodurch das Vertriebsglied 8 zumindest ein sehr kleines Stück in die proximale Richtung bewegt wird. Hierdurch wird der Kolben des Produktbehälters 14 entlastet und die Feder 11 zumindest minimal gespannt.

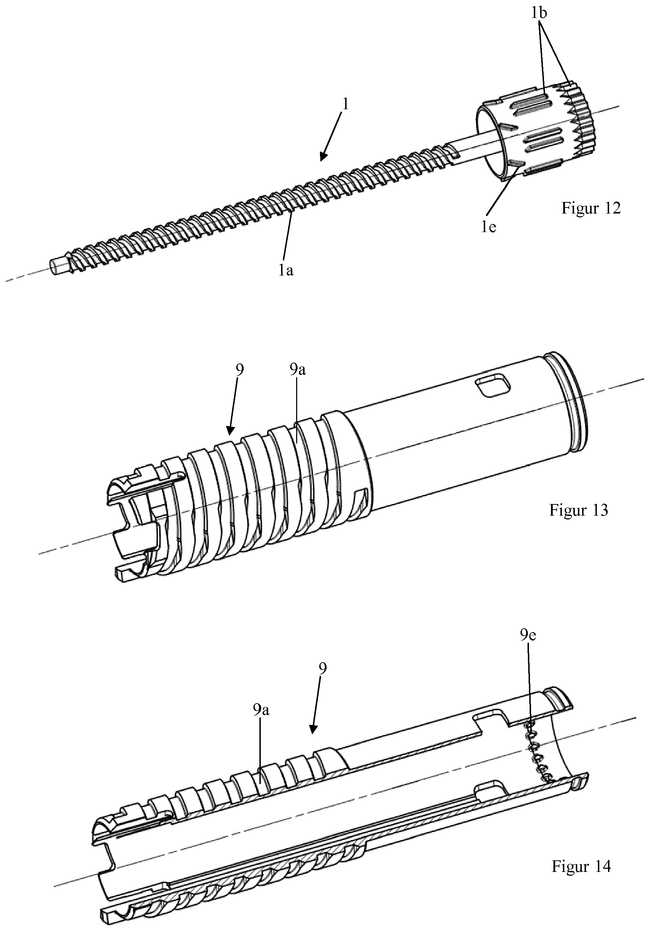

Die zweite Ausführungsform aus den Figuren 10 bis 14 umfasst eine als Innenverzahnung ausgestaltete erste Kupplungsstruktur 9e am Innenumfang des Lagerelements 9 (Figur 14). Das Lagerelement 9 ist relativ zu dem Gehäuse 4 drehfest und axial verschiebbar, wobei die Innenhülse 4a parallel zur Längsachse L sich erstreckende Stege aufweist, die in korrespondierende Ausnehmungen oder Nuten am Innenumfang des Lagerelements 9 (Figur 14) eingreifen. Das Rotationsglied 1 weist, wie beschrieben, eine zweite Kupplungsstruktur 1b, die als Außenverzahnung gebildet ist, auf. Ferner weist das Rotationsglied 1 wenigstens eine, vorzugsweise mehrere Getriebeflächen 1e auf, welche in Bezug auf die Längsachse L schräg angeordnet sind und an denen die erste Kupplungsstruktur 9e beim Betätigen des Betätigungsglieds 7 bzw. bei der Verschiebung des Lagerelements 9 in die distale Richtung relativ zu dem Gehäuse 4 abgleitet, wodurch das Rotationsglied 1 in eine Drehbewegung versetzt wird, wenn das Betätigungsglied 7 aus seiner Ausgangsposition in die betätigte Position verschoben wird. Hierbei wird die erste Kupplungsstruktur 9e des Lagerelements 9 aus dem Eingriff mit der zweiten Kupplungsstruktur 1b und in den Eingriff mit der Getriebefläche 1e bewegt. Die mindestens eine Getriebefläche 1e wird von mindestens einer Abragung, die vorzugsweise radial nach außen weist, gebildet. Die Abragung 1e kann rippenförmig sein und vorzugsweise distal der zweiten Kupplungsstruktur 1b angeordnet sein.

Die zweite Kupplungsstruktur 1b, die erste Kupplungsstruktur 9e und die mindestens eine Getriebefläche 1e können so aufeinander abgestimmt sein, dass die erste Kupplungsstruktur 9e noch in dem Eingriff mit der zweiten Kupplungsstruktur 1b ist, wenn die erste Kupplungsstruktur 9e an der Getriebefläche 1e abgleitet, oder aus dem Eingriff mit der zweiten Kupplungsstruktur 1b ist, wenn die Kupplungsstruktur 9e an der mindestens einen Getriebefläche 1e abgleitet.

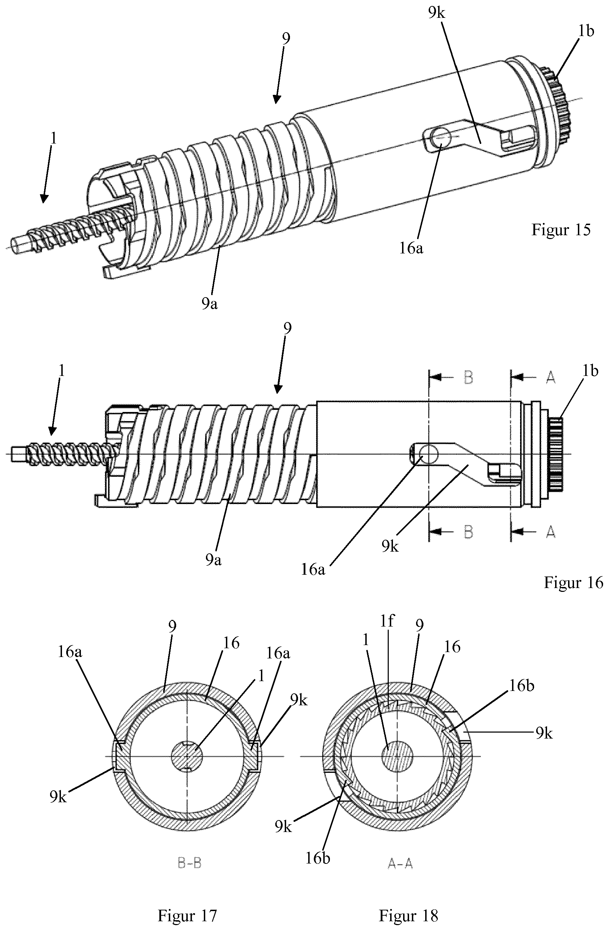

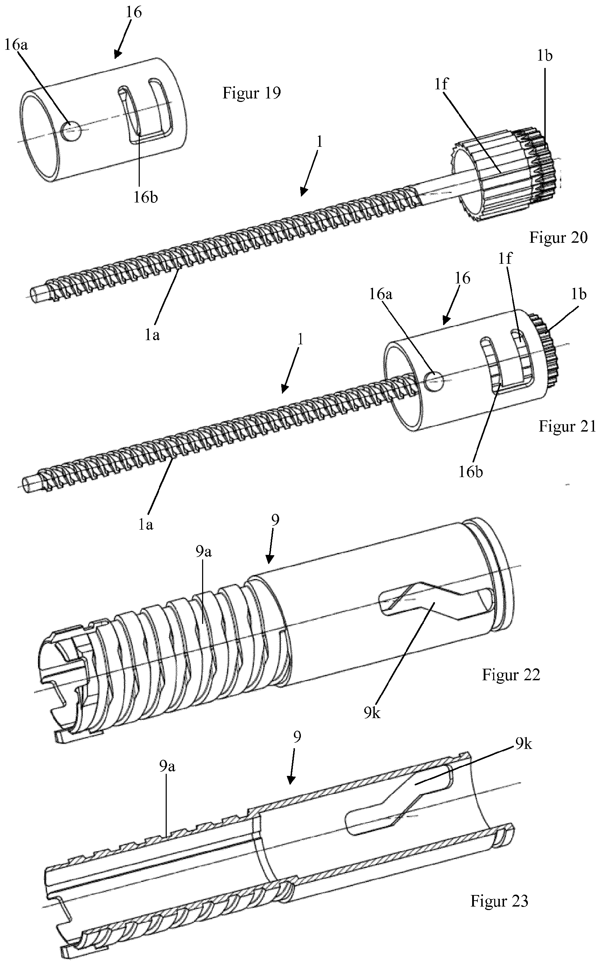

In der in den Figuren 15 bis 23 gezeigten dritten Ausführungsform ist zwischen dem Rotationsglied 1 und dem Lagerelement 9 eine Zwischenhülse 16 angeordnet, insbesondere geometrisch und/oder kinematisch. Die Zwischenhülse 16 weist eine federnd angeordnete Rastnocke 16b auf, die in eine Außenverzahnung des Rotationsglieds 1 federnd eingreift, wobei die Außenverzahnung eine Vielzahl über den Umfang des Rotationsglieds 1 angeordnete sägezahnförmige Zähne aufweist. Die Außenverzahnung ist am Außenumfang des Rotationsglieds 1 gebildet. Durch die sägezahnförmige Form der Außenverzahnung wird bewirkt, dass das Rotationsglied 1 bei der Drehung der Zwischenhülse 16 in die erste Drehrichtung, welche eine Produktausschüttung bewirkt, mitgenommen wird und dass das Rotationsglied 1 relativ zu der Zwischenhülse 16 in die erste Drehrichtung, welche die Ausschüttung des Produkts bewirkt, gedreht werden kann.

Die Zwischenhülse 16 weist eine Führungsnocke 16a auf, die am Außenumfang der Zwischenhülse 16 gebildet ist und radial nach außen, d. h. von der Längsachse L weg abragt. Die Führungsnocke 16a greift in eine nutförmige Führungsbahn 9k ein, welche von dem hülsenförmigen Lagerelement 9 gebildet wird. Die Führungsbahn 9k ist zumindest zum Innenumfang oder zur Zwischenhülse 16 hin offen. Die Führungsbahn 9k weist einen proximalen Führungsbahnabschnitt und einen distalen Führungsbahnabschnitt auf, die über einen Zwischenabschnitt, der schräg zu den distalen und proximalen Führungsbahnabschnitten angeordnet ist, miteinander verbunden sind. Die proximalen und distalen Führungsbahnabschnitte sind parallel zur Längsachse L und in Bezug zueinander um die Längsachse L winkelversetzt.

In der unbetätigten Position des Betätigungsglieds 7 ist die Führungsnocke 16a in dem distalen Führungsbahnabschnitt der Führungsbahn 9k angeordnet. Wenn das Betätigungsglied 7 in die betätigte Position gedrückt wird, wird das verdrehfest und axial verschiebbar mit dem Gehäuse 4 verbundene Lagerelement 9 in die distale Richtung relativ zu der Führungsnocke 16a bzw. zu der Zwischenhülse 16 und insbesondere relativ zu dem Gehäuse 4 verschoben, wodurch die Führungsnocke 16a in den Eingriff mit dem Zwischenabschnitt der Führungsbahn 9k gelangt, so dass die Führungsnocke 16a und die Zwischenhülse 16 in die erste Drehrichtung gedreht werden, wodurch das Rotationsglied 1 von der Zwischenhülse 16 in die erste Drehrichtung mitgenommen wird. Die Rastnocke 16b wird in der Verzahnung 1f gehalten, insbesondere durch den Innenumfang des Lagerelements 9. Wenn das Betätigungsglied 7 in seiner betätigten Position ist, ist der proximale Führungsbahnabschnitt der Führungsbahn 9k radial über der Rastnocke 16b, so dass die Rastnocke 16b radial nach außen federn kann (Figur 18a). Durch die Antriebsfeder 11 und die geschlossene Kupplung 1b, 2d wird das Rotationsglied 1 relativ zu der Zwischenhülse 16 in die erste Drehrichtung gedreht, so dass das Vortriebsglied 8 in die distale Richtung bewegt wird. Während der Drehung des Rotationsglieds 1 relativ zu der Zwischenhülse 16 rastet die Verzahnung 1f an der Rastnocke 16b entlang, so dass die Rastnocke 16b federnd aus dem und in den Eingriff mit der Verzahnung 1f gedrückt wird.

In den Ausführungen, in denen die Rastnocke 16b von dem Innenumfang des Lagerelements 9 in dem Eingriff mit der Verzahnung 1f gehalten wird, wenn das Betätigungsglied 7 nicht in seiner betätigten Position ist, braucht die Verzahnung 1f nicht notwendigerweise mit sägezahnförmigen Zähnen sondern kann allgemein mit Zähnen ausgestattet sein, da der Eingriff der Rastnocke 16b in die Verzahnung 1f bei der Drehung der Zwischenhülse 16 in die erste Drehrichtung dadurch bewirkt wird, dass der Innenumfang des Lagerelements 9 die Rastnocke 16b in dem Eingriff mit der Verzahnung 1f hält, so dass dieser Eingriff nicht lösbar ist, solange das Betätigungsglied 7 nicht in seiner Betätigungsposition ist.

In den ersten bis dritten Ausführungsformen kann das Lagerelement 9 alternativ als Verschiebeglied 9 bezeichnet werden, da es die Aufgabe des hierin beschriebenen Verschiebeglieds erfüllt.

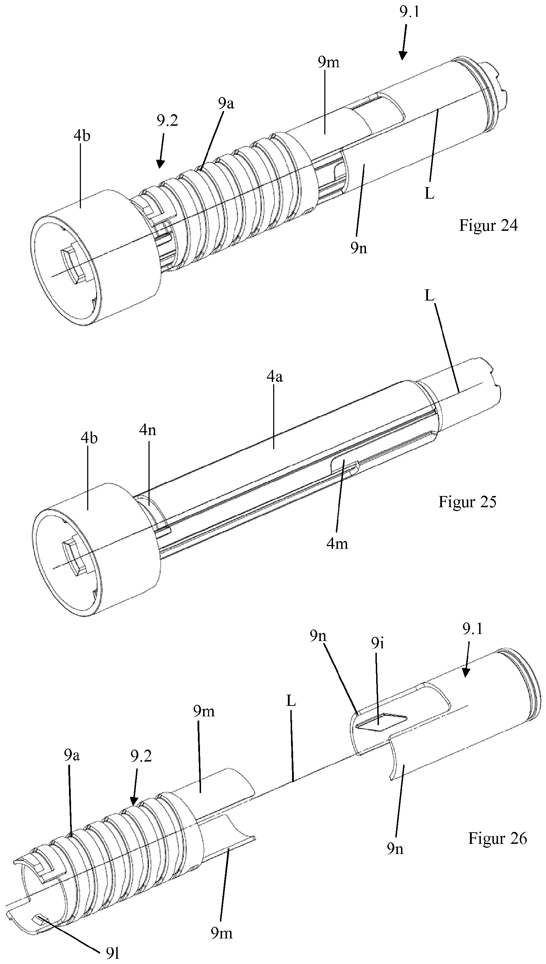

Die in den Figuren 24 bis 26 dargestellte vierte Ausführungsform löst die Aufgabe, eine Verschiebung der Dosisanzeigetrommel 10 entlang der Längsachse L relativ zu der Zeigeeinrichtung 4d und/oder dem Gehäuse 4 während des Verschiebens des Betätigungsglieds 7 zu verhindern. Für manche Benutzer oder Anwendungen kann es von Vorteil sein, wenn die Anzeige der Dosis durch Drücken des Betätigungsglieds 7 nicht beeinflusst wird, solange das Befestigungsglied 7 seine betätigte Position noch nicht erreicht hat. Durch die in den Figuren 24 und 26 gezeigte Ausführungsform lässt sich die in den Figuren 1 bis 5c gezeigte Grundkonstruktion für die Lösung dieser Angabe modifizieren. In den Figuren 24 bis 26 werden nur die Teile gezeigt, die zum Verständnis der Modifikation erforderlich sind und die insbesondere geändert sind.

In den Figuren 24 und 25 wird ein Abschnitt der Außenhülse 4b gezeigt, der über radiale Stege (nicht sichtbar, da durch den Abschnitt der Außenhülse 4b verdeckt) mit der Innenhülse 4a verbunden sind. Die Innenhülse 4a weist analog zu der Ausführung aus den Figuren 6 bis 9 eine nutförmige Führungsbahn 4m auf. Für die Ausgestaltung der Führungsbahn 4m wird auf die Beschreibung zu den Figuren 6 bis 9 verwiesen. Es wird jedoch darauf hingewiesen, dass der Übergangsbereich zwischen dem proximalen Führungsbahnabschnitt und dem distalen Führungsbahnabschnitt in den Figuren 24 bis 26 etwas weiter in proximale Richtung versetzt ist, da die Abragung 9i ebenfalls etwas weiter in proximale Richtung angeordnet ist als bei der Ausführungsform aus den Figuren 6 bis 9.

Das Lagerelement 9 umfasst in dieser Ausführungsform zwei Teile, nämlich ein erstes Lagerelementteil 9.1 und ein zweites Lagerelementteil 9.2. Das Lagerelementteil 9.1 erfüllt insbesondere die Aufgabe des hierin beschriebenen Verschiebeglieds und kann optional als Verschiebeglied bezeichnet werden.

Das zweite Lagerelementteil 9.2 weist das Außengewinde 9a auf, in welches das Innengewinde des Dosisanzeigeelements 10 eingreift, so dass das Dosisanzeigeelement 10 an dem Außengewinde 9a schraubbar ist. Das zweite Lagerelementteil 9.2 und das Gehäuse, insbesondere die Innenhülse 4a greifen so ineinander, dass das Lagerelementteil 9.2 relativ zu dem Gehäuse 4 und um der Längsachse L drehbar und entlang der Längsachse L unverschiebbar ist. Das zweite Lagerelementteil 9.2 ist hülsenförmig und kann daher als zweite Lagerelementhülse bezeichnet werden.

Das erste Lagerelementteil 9.1 ist ebenfalls hülsenförmig und kann daher als erste Lagerelementhülse 9.1 bezeichnet werden. Das erste Lagerelementteil 9.1 ist axialfest mit dem Betätigungsglied 7 (siehe zum Beispiel Figur 2b) verbunden, so dass sich das erste Lagerelementteil 9.1 bei der Betätigung des Betätigungsglieds 7 mit diesem in die distale Richtung relativ zu dem Gehäuse 4 bewegt und beim Loslassen des Betätigungsglieds 7 relativ zu dem Gehäuse 4 in die proximale Richtung bewegt.

Das erste Lagerelementteil 9.1 weist - ähnlich wie das Verschiebeglied bzw. Lagerelement 9 aus den Figuren 6 bis 9 - an ihren Innenumfang eine nach innen gerichtete Abragung 9i auf, die als rautenförmige Nocke ausgestaltet ist. Die Abragung 9i greift in die Führungsbahn 4m ein, nämlich in den proximalen Führungsbahnabschnitt, wenn das Betätigungsglied 7 unbetätigt ist. Wird das Betätigungsglied 7 betätigt, d. h. um den Betätigungshub in die distale Richtung entlang der Längsachse L verschoben, wird auch die Abragung 9i in den Übergangsbereich verschoben. Die Funktion entspricht der aus den Figuren 6 bis 9, so dass auf die entsprechende Beschreibung verwiesen wird. Durch das Abgleiten der schrägen Fläche der Abragung 9i an der distalen Kante des Übergangsabschnitts wird das erste Lagerelementteil 9.1 um die Längsachse L verdreht, nämlich in die erste Drehrichtung. Wenn das Betätigungsglied 7 losgelassen wird, bewegt sich die Abragung 9i in die proximale Richtung, wobei die in die proximale Richtung weisende schräge Fläche an der anderen proximalen Kante des Übergangsbereichs abgleitet, so dass das erste Lagerelementteil 9.1 in die zweite Drehrichtung gedreht wird.

Das erste Lagerelementteil 9.1 weist ferner die erste Kupplungsstruktur 9e in der Gestalt einer Innenverzahnung auf (nicht sichtbar). Für die Ausgestaltung der ersten Kupplungsstruktur 9e wird analog auf die Beschreibung für die bzw. auf Figur 9 verwiesen.

Die erste Kupplungsstruktur 9e greift verdrehfest in die zweite Kupplungsstruktur 1b ein, wenn das Betätigungsglied 7 unbetätigt oder nicht vollständig betätigt ist. Während der Ausführung des Betätigungshubs des Betätigungsglieds 7 wird das erste Lagerelementteil 9.1 bereits dann gedreht, wenn die Kupplung 9e, 1b noch geschlossen ist. Hierdurch wird bewirkt, dass das Rotationsglied 1 mit dem ersten Lagerelementteil 9.1 mitgedreht wird. Die Funktion entspricht insofern der aus der Ausführung aus den Figuren 6 bis 9, weshalb ergänzend auf die entsprechenden Beschreibungsteile verwiesen wird.

Das erste Lagerelementteil 9.1 und das zweite Lagerelementteil 9.2 greifen axial verschiebbar und drehfest ineinander. Somit macht das zweite Lagerelementteil 9.2 die Drehung des ersten Lagerelementteils 9.1 mit. Das zweite Lagerelementteil 9.2 ist jedoch von der Axialbewegung des ersten Lagerelementteils 9.1 entkoppelt. Hierdurch wird bewirkt, dass das zweite Lagerelementteil 9.2 entlang der Längsachse L nicht verschoben wird, wodurch auch das Dosisanzeigeelement 10 - abgesehen vom Zurückdrehen während der Produktausschüttung - relativ zu der Zeigeeinrichtung 4d des Gehäuses 4 entlang der Längsachse L nicht verschoben wird.

In dem gezeigten Beispiel weist das erste Lagerelementteil 9.1 zwei in die distale Richtung gerichtete Schenkel 9n auf, zwischen die in Umfangsrichtung zwei Schenkel 9m eingreifen, die von dem zweiten Lagerelementteil 9.2 gebildet werden und von dessen proximalem Ende abragen. Die Schenkel 9m, 9n führen sich gegenseitig und verhindern dadurch, dass das erste Lagerelementteil 9.1 relativ zu dem zweiten Lagerelementteil 9.2 verdrehbar ist und erlauben es jedoch gleichzeitig, dass das erste Lagerelementteil 9.1 relativ zu dem zweiten Lagerelementteil 9.2 entlang der Längsachs L verschiebbar ist.

Für die axialfeste und verdrehbare Verbindung zwischen dem zweiten Lagerelementteil 9.2 und der Innenhülse 4a ist an der Innenhülse 4a eine sich in Umfangsrichtung erstreckende Nut 4n angeordnet, in welche ein Eingriffsglied 91, insbesondere ein Vorsprung am Innenumfang des zweiten Lagerelementteils 9.2 eingreift.

Das Kupplungsglied 2 (Figuren 1 bis 5c) greift axialfest und verdrehbar in das erste Lagerelementteil 9.1 ein, wofür das erste Lagerelementteil 9.1 zum Beispiel an seinem proximalen Ende einen ringförmig umlaufenden Kragen aufweist.

Die Kupplung 1b, 9e, die Kupplung 1b, 2d, die Nocke 9i und der Übergansbereich sind so aufeinander abgestimmt und insbesondere zueinander positioniert, dass während des Verschiebens des Betätigungsglieds 7 aus der unbetätigten Position in die betätigte Position erst die Kupplung 2d, 1b geschlossen, danach das Lagerelementteil 9.1 mittels der Nocke 9i verdreht und erst nach dem Beginn der Verdrehung des Lagerelementteils 9.1 die Kupplung 1b, 9e geöffnet werden.

Die Dosisanzeigetrommel 10 führt, insbesondere aufgrund der geschlossenen Kupplung 1b, 2d, während der Verdrehung des Lagerelementteils 9.1 eine Drehung, insbesondere ohne Bewegung entlang der Längsachse L, relativ zu der Zeigeeinrichtung 4d und/oder dem Gehäuse 4 aus. Währenddessen findet nämlich keine Drehbewegung und somit auch keine Schraubbewegung zwischen dem Lagerelementteil 9.2 und der Dosisanzeigetrommel 10 statt.

**Bezugszeichenliste**

| | | | |
|---|---|---|---|
| 1 | Rotationsglied / Gewindestange / Gewindemutter | 8 | Vortriebsglied / Stößel |
| | | 8a | Längsrippe |
| 1a | Außengewinde | 8b | Innenhülse |
| 1b | zweite Kupplungsstruktur / Zähne | 8c | Gewinde / Innengewinde |
| 1c | Verzahnung / Innenverzahnung | 8d | distales Ende |
| 1d | Rastglied | 8e | Gewindestange |
| 1e | Getriebefläche / Abragung | | |
| 1f | Zähne, insbesondere sägezahnförmig | 9 | Lagerelement / Verschiebeglied |
| | | 9a | Außengewinde |
| 2 | Kupplungsglied / Anzeigekupplung | 9b | Verzahnung / Außenverzahnung |
| 2a | Längsführung / Längsrippe | 9c | Maximaldosisgegenanschlag |
| 2b | Kupplungsstruktur | 9d | ringförmige Abragung |
| 2c | Ringnut | 9e | erste Kupplungsstruktur / Zähne |
| 2d | dritte Kupplungsstruktur | 9f | Längsführung |
| 2e | Rastglied | 9g | Rastglied |
| | | 9h | Verzahnung / Innenverzahnung |
| 3 | Dosierglied / Dosierknopf / Dosiseinstellglied | 9i | Abragung / rautenförmiger Nocken |
| | | 9k | Führungsbahn / Führungsnut |
| 3a | Längsführung | 9l | Eingriffsglied |
| 3b | Griffstruktur | 9m | Schenkel |
| 3c | Kupplungsstruktur | 9n | Schenkel |
| | | 9.1 | erstes Lagerelementteil |
| 4 | Gehäuse | 9.2 | zweites Lagerelementteil |
| 4a | Innenhülse | | |
| 4b | Außenhülse | 10 | Dosisanzeigeelement / |
| 4c | Längsführung | | Dosisanzeigetrommel |
| 4d | Zeigeeinrichtung / Fenster | 10a | Markierung |
| 4e | Gewinde / Außengewinde | 10b | Dosisskala |
| 4f | Nulldosisgegenanschlag | 10c | Nulldosisanschlag |
| 4g | Ringnut | 10d | Maximaldosisanschlag |
| 4h | Verzahnung / Stirnverzahnung / Innenverzahnung | 10e | Innengewinde |
| | | | |
| 4i | Widerlager | 11 | Feder / Ausschüttfeder |
| 4j | Begrenzerinnenhülse | 11a | erste Ausschüttfeder |
| 4k | Verbindungssteg | 11b | zweite Ausschüttfeder |
| 4l | Gewinde / Innengewinde | 11c | Zwischenglied |
| 4m | Führungsbahn / Führungsnut | | |
| 4n | Nut | 12 | Feder / Rücksetz- oder Kupplungsfeder |
| | | | |
| 5 | Produktbehälteraufnahme | | |
| | | 13 | Begrenzer / Dosisbegrenzer |
| 6 | Schutzkappe | 13a | Eingriffsglied |
| | | 13b | Gewinde / Innengewinde |
| 7 | Betätigungsglied / Betätigungsknopf | | |
| 7a | Verzahnung / Stirnverzahnung / Außenverzahnung | 14 | Produktbehälter / Karpule |
| | | 14a | Kolben |
| 14b | Septum | | |
| | | | |
| 16 | Zwischenhülse | | |
| 16a | Abragung / Führungsnocken | | |
| 16b | Eingriffsglied / Rastnocken | | |
| | | | |
| L | Längsachse / Drehachse | | |

## Patentansprüche

1. Antriebsvorrichtung, insbesondere Antriebs- und Dosiervorrichtung, für eine Injektionsvorrichtung zur Verabreichung eines flüssigen Produkts, insbesondere eines Medikaments, umfassend:
a) ein Gehäuse (4),
b) ein insbesondere vom Verwender der Antriebsvorrichtung betätigbares Betätigungsglied (7), das bei Betätigung aus einer Ausgangsposition zur Ausschüttung des Produkts relativ zu dem Gehäuse (4) verschiebbar ist oder verschoben wird,
c) ein Vortriebsglied (8), dessen distales Ende (8d) vorgesehen ist, auf einen Kolben (14a) eines an der Antriebsvorrichtung befestigten oder befestigbaren Produktbehälters (14) zu wirken,
d) ein Widerlager (4i), wobei das Vortriebsglied (8) relativ zu dem Widerlager (4i) in eine Ausschüttrichtung bewegbar ist, um eine Ausschüttung des Produkts zu bewirken,
e) eine zwischen dem Vortriebsglied (8) und dem Widerlager (4i) wirkende und im Auslieferungszustand der Vorrichtung vorgespannte Feder (11), die mit so viel Energie vorgespannt ist, dass sie die aus dem Produktbehälter (14) maximal ausschüttbare Produktmenge ausschütten kann,
f) ein Rotationsglied (1), dessen Drehung relativ zu dem Gehäuse (4) bewirkt, dass die Feder (11) das Vortriebsglied (8) in Ausschüttrichtung bewegen kann,
g) und eine Kupplung (9e, 1b), die geschlossen ist und durch Betätigen, insbesondere Drücken, des Betätigungsglieds (7) geöffnet wird, wobei die geöffnete Kupplung (9e, 1b) die Drehung des Rotationsglieds (1) relativ zu dem Gehäuse (4), insbesondere in eine erste Drehrichtung, freigibt,
**dadurch gekennzeichnet, dass**
h) das Betätigungsglied (7) so mit dem Rotationsglied (1) gekoppelt ist, dass das Betätigen, insbesondere Drücken, des Betätigungsglieds (7) bewirkt, dass sich das Rotationsglied (1) relativ zu dem Gehäuse (4) bereits dann dreht, wenn die Kupplung (9e, 1b) noch geschlossen ist.

2. Antriebsvorrichtung nach Anspruch 1, **dadurch gekennzeichnet, dass** die durch das Betätigen des Betätigungsglieds (7) bewirkte Drehung des Rotationsglieds (1) relativ zu dem Gehäuse (4) bei geschlossener Kupplung (9e, 1b) kleiner als 45°, insbesondere kleiner als 20° und/oder in die erste oder die gleiche Drehrichtung gerichtet ist, wie die Drehrichtung der mittels der vorgespannten Feder bewirkbare Drehung des Rotationsglieds (1).

3. Antriebsvorrichtung nach einem der vorhergehenden Ansprüche, **dadurch gekennzeichnet, dass** das Betätigungsglied (7) so mit dem Rotationsglied (1) gekoppelt ist, dass das Zurückbewegen des Betätigungsglieds (7) aus seiner betätigten Position in seine Ausgangsposition, insbesondere das Loslassen des Betätigungsglieds (7), bewirkt, dass sich das Rotationsglied (1) relativ zu dem Gehäuse (4) in eine Drehrichtung, insbesondere eine zweite Drehrichtung, dreht, die der Drehrichtung, insbesondere der ersten Drehrichtung, der mittels der vorgespannten Feder (11) bewirkbaren Drehung entgegengesetzt ist.

4. Antriebsvorrichtung nach einem der vorhergehenden Ansprüche, **dadurch gekennzeichnet, dass** der Kolben (14a) des Produktbehälters (5) bei der Produktausschüttung oder bei der Drehung des Rotationsglieds (1) in die erste Drehrichtung von dem auf ihn in die Ausschüttrichtung wirkenden Vortriebsglied (8) in Ausschüttrichtung verschoben wird und dass beim Zurückbewegen des Betätigungsglieds (7) in seine Ausgangsposition das Vortriebsglied (8) entgegen die Ausschüttrichtung relativ zu dem Widerlager (4i) bewegt wird, insbesondere zumindest so weit, dass der Kolben (14a) von dem Druck des Vortriebsglieds (8) entlastet wird.

5. Antriebsvorrichtung nach einem der vorhergehenden Ansprüche, **dadurch gekennzeichnet, dass** ein erstes Teil ein erstes Getriebeelement und ein zweites Teil ein zweites Getriebeelement bilden, wobei das erste Getriebeelement und das zweite Getriebeelement durch die Betätigung des Betätigungsglieds (7) aneinander abgleiten, wodurch das Betätigen, insbesondere das Drücken des Betätigungsglieds (7) bewirkt, dass sich das Rotationsglied relativ zu dem Gehäuse bereits dann dreht, wenn die Kupplung (9e, 1b) noch geschlossen ist.

6. Antriebsvorrichtung nach dem vorhergehenden Anspruch, **dadurch gekennzeichnet, dass**
- das Gehäuse (4) oder ein gehäusefestes Element (4a) das erste Teil und ein die Kupplung (9e, 1b) mitbildendes Verschiebeglied (9), insbesondere ein Lagerelement, welches axial verschiebbar in Bezug auf das Gehäuse (4) oder das gehäusefeste Element (4a) ist, das zweite Teil ist;
oder
- das die Kupplung (9e, 1b) mitbildende Rotationsglied (1) das erste Teil und ein die Kupplung (9e, 1b) mitbildendes Verschiebeglied (9), insbesondere ein Lagerelement, welches drehfest und axial verschiebbar in Bezug auf das Gehäuse (4) oder ein gehäusefestes Element (4a) ist, das zweite Teil ist;
oder
- das Rotationsglied (1) eine Vielzahl über seinen Umfang angeordnete z. B. sägezahnförmige Zähne (1f) aufweist, in die eine Rastnocke (16b), die von einer um den Umfang des Rotationsglieds (1) angeordneten Hülse (16) gebildet ist, federnd eingreift, wobei die Hülse (16) das erste Teil ist und eine Kupplungsstruktur (1b) der Kupplung (9e, 1b) bildet und wobei ein Verschiebeglied (9), insbesondere ein Lagerelement, welches drehfest und axial verschiebbar in Bezug auf das Gehäuse (4) oder ein gehäusefestes Element (4a) ist, das zweite Teil ist, wobei das bevorzugt hülsenförmige Verschiebeglied (9) die Hülse (16) über ihren Umfang umgibt.

7. Antriebsvorrichtung nach einem der vorhergehenden Ansprüche, wobei die Antriebsvorrichtung eine Antriebs- und Dosiervorrichtung ist, mit der wiederholt zu verabreichende Produktdosen einstellbar sind, **gekennzeichnet durch** ein insbesondere vom Verwender der Antriebs- und Dosiervorrichtung greifbares Dosierglied (3), welches zur Einstellung einer zu verabreichenden Produktdosis relativ zu dem Gehäuse (4) drehbar ist oder gedreht wird, wobei das Betätigungsglied (7) zur Ausschüttung der eingestellten Produktdosis relativ zu dem Dosierglied (3) verschiebbar ist oder verschoben wird, wobei das Vortriebsglied (8) relativ zu dem Widerlager (4i) in die Ausschüttrichtung bewegbar ist, um eine Ausschüttung der eingestellten Produktdosis zu bewirken und wobei die im Auslieferungszustand der Vorrichtung vorgespannte Feder (11) mit so viel Energie vorgespannt ist, dass sie die aus dem Produktbehälter (14) ausschüttbare Produktmenge in mehreren Einzelausschüttungen ausschütten kann.

8. Antriebsvorrichtung nach einem der vorhergehenden Ansprüche, **dadurch gekennzeichnet, dass** die Kupplung (9e, 1b) durch Loslassen des Betätigungsglieds (7) die Drehung des Rotationsglieds (1) relativ zu dem Gehäuse (4) blockiert.

9. Antriebsvorrichtung nach Anspruch 1, **dadurch gekennzeichnet, dass** die Feder (11) mindestens eine Druckfeder ist, die sich insbesondere an dem Vortriebsglied (8) und dem Widerlager (4i) abstützt, oder eine Torsionsfeder ist, die sich an dem Rotationsglied (1) und dem Widerlager (4i) abstützt.

10. Antriebsvorrichtung nach einem der zwei vorhergehenden Ansprüche **dadurch gekennzeichnet, dass** das Widerlager (4i) durch das Gehäuse (4) oder einem gehäusefesten Element gebildet wird und/oder dass das Vortriebsglied (8) an dem Gehäuse (4) oder dem gehäusefesten Element geführt ist.

11. Antriebsvorrichtung nach einem der drei vorhergehenden Ansprüche, **dadurch gekennzeichnet, dass** die Feder (11) das Vortriebsglied (8) antreibt und das Vortriebsglied (8) das Rotationsglied (1) antreibt.

12. Antriebsvorrichtung nach einem der vorhergehenden Ansprüche, **dadurch gekennzeichnet, dass** das Rotationsglied (1) eine Gewindestange und das Vortriebsglied (8) eine Gewindemutter aufweisen und das Gewinde der Gewindemutter in das Gewinde der Gewindestange eingreift, oder dass das Rotationsglied (1) eine Gewindemutter und das Vortriebsglied (8) eine Gewindestange aufweisen und das Gewinde der Gewindemutter in das Gewinde der Gewindestange eingreift.

13. Antriebsvorrichtung nach einem der vorhergehenden Ansprüche, **dadurch gekennzeichnet, dass** ein Dosisanzeigeelement (10) einen Anschlag (10c) aufweist, der von einem Gegenanschlag (4f) wegbewegt wird, wenn eine Dosiserhöhung vorgenommen wird, und der zu dem Gegenanschlag (4f) hinbewegt wird, wenn eine Dosisverringerung vorgenommen wird oder wenn die Vorrichtung, insbesondere das Betätigungsglied (7) für die Ausschüttung der eingestellten Produktdosis betätigt ist.

14. Antriebsvorrichtung nach einem der vorhergehenden Ansprüche, **dadurch gekennzeichnet, dass** das Dosisanzeigeelement (10) während des Einstellens der Produktdosis, d. h. bei Dosiserhöhung und Dosisverringerung, von dem Rotationsglied (1) zumindest rotatorisch entkoppelt ist und bei der Betätigung der Vorrichtung, insbesondere des Betätigungsglieds (7), zum Ausschütten der Produktdosis so mit dem Rotationsglied (1) gekoppelt ist, dass eine Drehung des Rotationsglieds (1) bewirkt, dass das Dosisanzeigeelement (10) zu dem Gegenanschlag (4f) hinbewegt wird.

15. Antriebsvorrichtung nach einem der vorhergehenden Ansprüche, **dadurch gekennzeichnet, dass** das Dosierglied (3) zumindest bei der Dosiseinstellung rotatorisch von der Feder (11) entkoppelt ist, so dass die Feder (11) bei der Dosiseinstellung weder gespannt noch entspannt wird.

## Claims

1. A drive device, in particular a drive and metering device, for an injection device for administering a liquid product, in particular a drug, comprising:
a) a housing (4),
b) an actuating member (7) which is actuable in particular by the user of the drive device and which upon actuation is displaced or is displaceable from a starting position for dispensing the product relative to the housing (4),
c) an advance member (8) whose distal end (8d) is provided to act on a piston (14a) of a product container (14) which is fixed or can be fixed in the drive device,
d) an abutment (4i), wherein the advance member (8) is moveable relative to the abutment (4i) in a dispensing direction to cause dispensing of the product,
e) a spring which acts between the advance member (8) and the abutment (4i) and which is prestressed in the delivery state of the device and which is prestressed with so much energy that it can dispense the maximum amount of product which can be dispensed from the product container (14),
f) a rotary member (1), the rotation of which relative to the housing (4) provides that the spring (11) can move the advance member (8) in the dispensing direction, and
g) a coupling means (9e, 1b) which is closed and is opened by actuation, in particular pressing, of the actuating member (7), wherein the opened coupling means (9e, 1b) enables rotation of the rotary member (1) relative to the housing (4), in particular in a first direction of rotation,
**characterised in that**
h) the actuating member (7) is so coupled to the rotary member (1) that actuation, in particular pressing, of the actuating member (7) causes the rotary member (1) to already rotate relative to the housing (4) when the coupling means (9e, 1b) is still closed.

2. A drive device according to claim 1 **characterised in that** the rotation of the rotary member (1) caused by actuation of the actuating member (7) relative to the housing (4) when the coupling means (9e, 1b) is closed is less than 45°, in particular less than 20° and/or is directed in the first direction of rotation or the same direction of rotation as the direction of rotation of the rotation of the rotary member (1) which can be caused by means of the prestressed spring.

3. A drive device according to one of the preceding claims **characterised in that** the actuating member (7) is so coupled to the rotary member (1) that the return movement of the actuating member (7) from its actuated position into its starting position, in particular release of the actuating member (7), causes the rotary member (1) to rotate relative to the housing (4) in a direction of rotation, in particular a second direction of rotation, which is opposite to the direction of rotation, in particular the first direction of rotation, of the rotation which can be caused by means of the prestressed spring (11).

4. A drive device according to one of the preceding claims **characterised in that** the piston (14a) of the product container (5) upon product dispensing or upon rotation of the rotary member (1) in the first direction of rotation is displaced in the dispensing direction by the advance member (8) acting therein in the dispensing direction and that when the actuating member (7) moves back into its starting position the advance member (8) is moved in opposite relationship to the dispensing direction relative to the abutment (4i), in particular at least to such an extent that the piston (14a) is relieved of the pressure of the advance member (8).

5. A drive device according to one of the preceding claims **characterised in that** a first part forms a first transmission element and a second part forms a second transmission element, wherein the first transmission element and the second transmission element slide against each other by actuation of the actuating member (7) whereby actuation, in particular pressing, of the actuating member (7) causes the rotary member to already rotate relative to the housing when the coupling means (9e, 1b) is still closed.

6. A drive device according to the preceding claim **characterised in that**
- the housing (4) or an element (4a) fixed with respect to the housing is the first part and a displacement member (9), in particular a bearing element, which also forms the coupling means (9e, 1b) and which is axially displaceable in relation to the housing (4) or the element (4a) fixed with respect to the housing is the second part; or
- the rotary member (1) which also forms the coupling means (9e, 1b) is the first part and a dispplacement member (9), in particular a bearing element, which also forms the coupling means (9e, 1b) and which is non-rotatable and axially displaceable in relation to the housing (4) or an element (4a) fixed with respect to the housing is the second part; or
- the rotary member (1) has a plurality of for example sawtooth-shaped teeth (1f) which are arranged over its periphery and into which a latching projection (16b) formed by a sleeve (16) arranged around the periphery of the rotary member (1) resiliently engages, wherein the sleeve (16) is the first part and forms a coupling structure (1b) of the coupling means (9e, 1b), and wherein a displacement member (9), in particular a bearing element, which is non-rotatable and axially displaceable in relation to the housing (4) or an element (4a) fixed with respect to the housing is the second part, wherein the preferably sleeve-shaped displacement member (9) surrounds the sleeve (16) over its periphery.

7. A drive device according to one of the preceding claims wherein the drive device is a drive and metering device with which product doses to be repeatedly administered are adjustable, **characterised by** a metering member (3) which can be gripped in particular by the user of the drive and metering device and which is rotated or is rotatable relative to the housing (4) to adjust a product dose to be administered, wherein the actuating member (7) is displaceable or is displaced for dispensing the adjusted product dose relative to the metering member (3), wherein the advance member (8) is moveable relative to the abutment (4i) in the dispensing direction to cause dispensing of the adjusted product dose and wherein the spring (11) which is prestressed in the delivery state of the device is prestressed with so much energy that it can dispense the amount of product which can be dispensed from the product container (14) in a plurality of individual dispensing quantities.

8. A drive device according to one of the preceding claims **characterised in that** the coupling means (9e, 1b) blocks the rotation of the rotary member (1) relative to the housing (4) by release of the actuating member (7).

9. A drive device according to claim 1 **characterised in that** the spring (11) is at least one compression spring which bears in particular against the advance member (8) and the abutment (4i) or a torsion spring which bears against the rotary member (1) and the abutment (4i).

10. A drive device according to one of two preceding claims **characterised in that** the abutment (4i) is formed by the housing (4) or an element fixed with respect to the housing and/or that the advance member (8) is guided at the housing (4) or the element fixed with respect to the housing.

11. A drive device according to one of the three preceding claims **characterised in that** the spring (11) drives the advance member (8) and the advance member (8) drives the rotary member (1).

12. A drive device according to one of the preceding claims **characterised in that** the rotary member (1) has a threaded rod and the advance member (8) has a threaded nut and the thread of the threaded nut engages into the thread of the threaded rod or the rotary member (1) has a threaded nut and the advance member (8) has a threaded rod and the thread of the threaded nut engages into the thread of the threaded rod.

13. A drive device according to one of the preceding claims **characterised in that** a dose indicating element (10) has an abutment portion (10c) which is moved away from a counterpart abutment portion (4f) when an increase in dose is effected and which is moved towards the counterpart abutment portion (4f) when a dose reduction is effected or when the device, in particular the actuating member (7), is actuated for dispensing the adjusted product dose.

14. A drive device according to one of the preceding claims **characterised in that** during adjustment of the product dose, that is to say upon dose increase and dose reduction, the dose indicating element (10) is at least rotationally uncoupled from the rotary member (1) and upon actuation of the device, in particular the actuating member (7), for dispensing the product dose is so coupled to the rotary member (1) that a rotation of the rotary member (1) causes the dose indicating element (10) to be moved towards the counterpart abutment portion (4f).

15. A drive device according to one of the preceding claims **characterised in that** at least in dose adjustment the metering member (3) is rotationally uncoupled from the spring (11) so that the spring (11) is neither stressed nor relieved of stress in the dose adjustment operation.

## Revendications

1. Dispositif d'entraînement, en particulier dispositif d'entraînement et de dosage, pour un dispositif d'injection pour l'administration d'un produit liquide, en particulier d'un médicament, comprenant :
a) un boîtier (4),
b) un organe d'actionnement (7) actionnable en particulier par l'utilisateur du dispositif d'entraînement qui est ou peut être déplacé lors de l'actionnement d'une position de départ pour le versement du produit par rapport au boîtier (4),
c) un organe de propulsion (8), dont l'extrémité distale (8d) est prévue afin d'agir sur un piston (14a) d'un récipient de produit (14) fixé ou pouvant être fixé sur le dispositif d'entraînement,
d) un contre-palier (4i), dans lequel l'organe de propulsion (8) est mobile par rapport au contre-palier (4i) dans un sens de versement afin de provoquer un versement du produit,
e) un ressort (11) agissant entre l'organe de propulsion (8) et le contre-palier (4i) et précontraint dans l'état de livraison du dispositif qui est précontraint avec tant d'énergie qu'il peut verser la quantité de produit versable au maximum hors du récipient de produit (14),
f) un organe de rotation (1), dont la rotation par rapport au boîtier (4) a pour effet que le ressort (11) peut déplacer l'organe de propulsion (8) dans le sens de versement,
g) et un couplage (9e, 1b) qui est fermé et est ouvert par actionnement, en particulier par pressage, de l'organe d'actionnement (7), dans lequel le couplage (9e, 1b) ouvert libère la rotation de l'organe de rotation (1) par rapport au boîtier (4), en particulier dans un premier sens de rotation,
**caractérisé en ce que**
h) l'organe d'actionnement (7) est couplé à l'organe de rotation (1) de sorte que l'actionnement, en particulier le pressage, de l'organe d'actionnement (7) ait pour effet que l'organe de rotation (1) tourne déjà par rapport au boîtier (4) lorsque le couplage (9e, 1b) est encore fermé.

2. Dispositif d'entraînement selon la revendication 1, **caractérisé en ce que** la rotation provoquée par l'actionnement de l'organe d'actionnement (7) de l'organe de rotation (1) par rapport au boîtier (4) en cas de couplage (9e, 1b) fermé est inférieure à 45°, en particulier inférieure à 20° et/ou est dirigée dans le premier ou le même sens de rotation que le sens de rotation de la rotation pouvant être provoquée à l'aide du ressort précontraint de l'organe de rotation (1).

3. Dispositif d'entraînement selon l'une des revendications précédentes, **caractérisé en ce que** l'organe d'actionnement (7) est couplé à l'organe de rotation (1) de sorte que le redéplacement de l'organe d'actionnement (7) de sa position actionnée à sa position de départ, en particulier le relâchement de l'organe d'actionnement (7) ait pour effet que l'organe de rotation (1) tourne par rapport au boîtier (4) dans un sens de rotation, en particulier un second sens de rotation qui est opposé au sens de rotation, en particulier au premier sens de rotation de la rotation pouvant être provoquée à l'aide du ressort (11) précontraint.

4. Dispositif d'entraînement selon l'une des revendications précédentes, **caractérisé en ce que** le piston (14a) du récipient de produit (5) est déplacé lors du versement de produit ou lors de la rotation de l'organe de rotation (1) dans le premier sens de rotation de l'organe de propulsion (8) agissant sur lui dans le sens de versement, dans le sens de versement et que lors du redéplacement de l'organe d'actionnement (7) dans sa position de départ, l'organe de propulsion (8) est déplacé dans le sens inverse au sens de versement par rapport au contre-palier (4i), en particulier au moins tant que le piston (14a) est déchargé de la pression de l'organe de propulsion (8).

5. Dispositif d'entraînement selon l'une des revendications précédentes, **caractérisé en ce qu'**une première partie forme un premier élément d'engrenage et une seconde partie forme un second élément d'engrenage, dans lequel le premier élément d'engrenage et le second élément d'engrenage glissent l'un sur l'autre par l'actionnement de l'organe d'actionnement (7), moyennant quoi l'actionnement, en particulier le pressage de l'organe d'actionnement (7) a pour effet que l'organe de rotation tourne déjà par rapport au boîtier lorsque le couplage (9e, 1b) est encore fermé.

6. Dispositif d'entraînement selon la revendication précédente, **caractérisé en ce que**
- le boîtier (4) ou un élément fixé au boîtier (4a) est la première partie et un organe de coulissement (9) formant le couplage (9e, 1b), en particulier un élément de palier qui est mobile axialement par rapport au boîtier (4) ou à l'élément fixé au boîtier (4a), est la seconde partie ;
ou
- l'organe de rotation (1) formant le couplage (9e, 1b) est la première partie et un organe de coulissement (9) formant le couplage (9e, 1b), en particulier un élément de palier qui est fixe en rotation et mobile axialement par rapport au boîtier (4) ou à un élément fixé au boîtier (4a), est la seconde partie ;
ou
- l'organe de rotation (1) présente une pluralité de dents (1f) par exemple en forme de dent de scie agencées sur sa périphérie, avec lesquelles une came d'encliquetage (16b), qui est formée par une douille (16) agencée autour de la périphérie de l'organe de rotation (1), vient en prise par ressort, dans lequel la douille (16) est la première partie et forme une structure de couplage (1b) du couplage (9e, 1b) et dans lequel un organe de coulissement (9), en particulier un élément de palier qui est fixe en rotation et mobile axialement par rapport au boîtier (4) ou à un élément fixé au boîtier (4a), est la seconde partie, dans lequel l'organe de coulissement (9) de préférence en forme de douille entoure la douille (16) sur sa périphérie.

7. Dispositif d'entraînement selon l'une des revendications précédentes, dans lequel le dispositif d'entraînement est un dispositif d'entraînement et de dosage, avec lequel des doses de produit à administrer de manière répétée sont réglables, **caractérisé par** un organe de dosage (3) pouvant être saisi en particulier par l'utilisateur du dispositif d'entraînement et de dosage, qui est rotatif ou est tourné pour le réglage d'une dose de produit à administrer par rapport au boîtier (4), dans lequel l'organe d'actionnement (7) est mobile ou est déplacé pour le versement de la dose de produit réglé par rapport à l'organe de dosage (3), dans lequel l'organe de propulsion (8) est mobile par rapport au contre-palier (4i) dans le sens de versement afin de provoquer un versement de la dose de produit réglée et dans lequel le ressort (11) précontraint dans l'état de livraison du dispositif est précontraint avec tant d'énergie qu'il peut verser la quantité de produit versable du récipient de produit (14) en plusieurs versements individuels.

8. Dispositif d'entraînement selon l'une des revendications précédentes, **caractérisé en ce que** le couplage (9e, 1b) bloque par relâchement de l'organe d'actionnement (7) la rotation de l'organe de rotation (1) par rapport au boîtier (4).

9. Dispositif d'entraînement selon la revendication 1, **caractérisé en ce que** le ressort (11) est au moins un ressort de pression qui s'appuie en particulier contre l'organe de propulsion (8) et le contre-palier (4i) ou est un ressort de torsion qui s'appuie contre l'organe de rotation (1) et le contre-palier (4i).

10. Dispositif d'entraînement selon l'une des deux revendications précédentes, **caractérisé en ce que** le contre-palier (4i) est formé par le boîtier (4) ou un élément fixé au boîtier et/ou que l'organe de propulsion (8) est guidé sur le boîtier (4) ou l'élément fixé au boîtier.

11. Dispositif d'entraînement selon l'une des trois revendications précédentes, **caractérisé en ce que** le ressort (11) entraîne l'organe de propulsion (8) et l'organe de propulsion (8) entraîne l'organe de rotation (1).

12. Dispositif d'entraînement selon l'une des revendications précédentes, **caractérisé en ce que** l'organe de rotation (1) présente une tige filetée et l'organe de propulsion (8) présente un écrou fileté et le filet de l'écrou fileté vient en prise avec le filet de la tige filetée ou que l'organe de rotation (1) présente un écrou fileté et l'organe de propulsion (8) présente une tige filetée et le filet de l'écrou fileté vient en prise avec le filet de la tige filetée.

13. Dispositif d'entraînement selon l'une des revendications précédentes, **caractérisé en ce qu'**un élément d'affichage de dose (10) présente une butée (10c) qui est déplacée loin d'une butée antagoniste (4f) si une augmentation de dose est entreprise, et qui est déplacé vers la butée antagoniste (4f) si une diminution de dose est entreprise ou si le dispositif en particulier l'organe d'actionnement (7) est actionné pour le versement de la dose de produit réglée.

14. Dispositif d'entraînement selon l'une des revendications précédentes, **caractérisé en ce que** l'élément d'affichage de dose (10) est découplé au moins en rotation de l'organe de rotation (1) pendant le réglage de la dose de produit, c'est-à-dire lors de l'augmentation de dose et de la diminution de dose et est couplé lors de l'actionnement du dispositif, en particulier de l'organe d'actionnement (7), pour le versement de la dose de produit avec l'organe de rotation (1) de sorte qu'une rotation de l'organe de rotation (1) ait pour effet que l'élément d'affichage de dose (10) est déplacé vers la butée antagoniste (4f).

15. Dispositif d'entraînement selon l'une des revendications précédentes, **caractérisé en ce que** l'organe de dosage (3) est découplé au moins lors du réglage de dose en rotation du ressort (11) de sorte que le ressort (11) ne soit ni tendu ni détendu lors du réglage de dose.
